(19) Europäisches Patentamt
European Patent Office
Office européen des brevets

(11) **EP 4 293 045 A1**

(12) **EUROPEAN PATENT APPLICATION**
published in accordance with Art. 153(4) EPC

(43) Date of publication:
**20.12.2023 Bulletin 2023/51**

(21) Application number: **22739121.6**

(22) Date of filing: **14.01.2022**

(51) International Patent Classification (IPC):
**C07K 16/28** (2006.01)      **A61P 35/00** (2006.01)
**A61K 39/395** (2006.01)      **C07K 16/18** (2006.01)

(52) Cooperative Patent Classification (CPC):
**A61K 9/08; A61K 9/19; A61K 39/395; A61K 47/02;**
**A61K 47/12; A61K 47/18; A61K 47/26;**
**A61P 35/00; C07K 16/18; C07K 16/28;** Y02A 50/30

(86) International application number:
**PCT/CN2022/072033**

(87) International publication number:
**WO 2022/152245 (21.07.2022 Gazette 2022/29)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **14.01.2021   CN 202110048207**

(71) Applicants:
• **Shanghai Junshi Biosciences Co., Ltd.**
  **Pilot Free Trade Zone**
  **Shanghai**
  **201210 (CN)**
• **Suzhou Junmeng Biosciences Co., Ltd.**
  **Jiangsu 215002 (CN)**

(72) Inventors:
• **LIU, Peixiang**
  **Jiangsu 215002 (CN)**
• **LIU, Hongchuan**
  **Jiangsu 215002 (CN)**
• **ZHANG, Jing**
  **Jiangsu 215002 (CN)**
• **ZHAO, Qiang**
  **Jiangsu 215002 (CN)**
• **FENG, Hui**
  **Jiangsu 215002 (CN)**

(74) Representative: **V.O.**
**P.O. Box 87930**
**2508 DH Den Haag (NL)**

Remarks:
The complete document including Reference Table(s) and the Sequence Listing(s) can be downloaded from the EPO website

(54) **ANTI-TIGIT ANTIBODY PHARMACEUTICAL COMPOSITION AND APPLICATION THEREOF**

(57)    A stable anti-TIGIT antibody pharmaceutical composition and an application thereof. The pharmaceutical composition comprises a buffer solution, and an anti-TIGIT antibody or an antigen-binding fragment thereof. The anti-TIGIT antibody or the antigen-binding fragment thereof comprises HCDR1, HCDR2, and HCDR3 having amino acid sequences as respectively represented by SEQ ID NO:1, SEQ ID NO:2, and SEQ ID NO:3, and LCDR1, LCDR2, and LCDR3 having amino acid sequences as respectively represented by SEQ ID NO:4, SEQ ID NO:5, and SEQ ID NO:6. The pH of the pharmaceutical composition is about 5.0-6.5.

EP 4 293 045 A1

**Description**

**Technical Field**

The present invention relates to the field of therapeutic pharmaceutical compositions, and specifically to an anti-TIGIT antibody pharmaceutical composition and an application thereof.

**Background Art**

TIGIT (T cell immunoreceptor with Ig and ITIM domains) is an immunomodulatory receptor that is composed of an extracellular immunoglobulin domain, a type I transmembrane region and two ITIM motifs and is mainly expressed on activated T cells and NK cells (Stanietsky et al., PNAS. 2009, 106, 17858-17863). Ligands recognized by TIGIT are a poliovirus receptor (PVR, CD155) and Nectin 2 (PVRL2/CD112), which are overexpressed on a variety of different tumour cells. It has been reported that TIGIT binds to ligand PVR with a high affinity. The binding of TIGIT to a ligand activates an inhibitory signal mediated by two motifs, namely an immunoreceptor tail tyrosine (ITT)-like motif and an immunoreceptor tyrosine-based inhibitory motif (ITIM), present in the cytoplasmic tail of TIGIT (Liu et al., Cell death and differentiation, 2013, 20, 456-464; Stanietsky et al., European journal of immunology, 2013, 43, 2138-2150). Ligands on the surface of tumour cells inhibit NK cytotoxicity and T cell activity by binding to the ITIM domain of TIGIT on the surface of NK cells and T cells, thereby mediating the immune evasion mechanism of tumour cells. A number of patent documents have reported that anti-TIGIT antibodies capable of blocking the binding of TIGIT to its ligands can be used in the treatment of diseases such as tumours by inhibiting TIGIT-mediated immunosuppression (WO 2004/024068, WO 2009/126688, WO 2015/009856, and WO 2016/028656).

There is an unmet need to provide other more effective, specific, safe and/or stable agents capable of enabling cells of the immune system to attack tumour cells by reducing the inhibitory activity of human TIGIT, either alone or in combination with other agents.

**Summary of the Invention**

The pharmaceutical composition of the present invention is a highly stable pharmaceutical composition comprising a humanized antibody that specifically binds to TIGIT. In particular, the present invention finds that trehalose can obviously improve the stability of the pharmaceutical composition.

The present invention provides a pharmaceutical composition, comprising: (1) a buffer solution; and (2) an anti-TIGIT antibody or an antigen-binding fragment thereof.

In some embodiments, the pH of the above-mentioned pharmaceutical composition is about 5.0-6.5, preferably about 5.0-6.0, and more preferably about 5.5.

In some embodiments, the above-mentioned anti-TIGIT antibody or antigen-binding fragment thereof comprises HCDR1, HCDR2, and HCDR3 having amino acid sequences as respectively represented by SEQ ID NO:1, SEQ ID NO:2, and SEQ ID NO:3, and LCDR1, LCDR2, and LCDR3 having amino acid sequences as respectively represented by SEQ ID NO:4, SEQ ID NO:5, and SEQ ID NO:6.

In some embodiments, the anti-TIGIT antibody or the antigen-binding fragment thereof in the pharmaceutical composition has a concentration of about 1-300 mg/mL, preferably about 1-250 mg/mL, preferably about 1-200 mg/mL, preferably about 10-80 mg/mL, and more preferably 10-40 mg/mL. More preferably, the above-mentioned anti-TIGIT antibody or antigen-binding fragment thereof has a concentration of about 5 mg/mL, 10 mg/mL, 15 mg/mL, 20 mg/mL, 25 mg/mL, 30 mg/mL, 35 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL or 140 mg/mL, preferably about 10 mg/mL, 20 mg/mL or 40 mg/mL.

In some embodiments, the above-mentioned buffer solution is selected from one or more of an acetate buffer, a citrate buffer and a histidine buffer; preferably, the buffer solution is an acetate buffer.

In some embodiments, the above-mentioned buffer solution is a histidine buffer; preferably, the histidine buffer is selected from a histidine-hydrochloride buffer or a histidine-acetate buffer, preferably a histidine-hydrochloride buffer.

In some embodiments, the above-mentioned histidine buffer is a histidine-hydrochloride buffer. In some embodiments, the above-mentioned histidine-hydrochloride buffer is made from histidine and histidine hydrochloride, preferably L-histidine and L-histidine monohydrochloride. In some embodiments, the histidine buffer is made from 1-30 mM L-histidine and 1-30 mM L-histidine monohydrochloride. In some embodiments, the histidine buffer is made from histidine and histidine hydrochloride in a molar ratio of 1 : 1 to 1 : 4. In some embodiments, the histidine buffer is made from histidine and histidine hydrochloride in a molar ratio of 1 : 1. In some embodiments, the histidine buffer is made from histidine and histidine hydrochloride in a molar ratio of 1 : 3. In some embodiments, a histidine preparation is: a histidine buffer with pH 5.5 made from 4.5 mM L-histidine and 15.5 mM L-histidine monohydrochloride. In some embodiments, a histidine preparation is: a histidine buffer with pH 5.5 made from 7.5 mM L-histidine and 22.5 mM L-histidine monohydrochloride. In some embodiments, a histidine preparation is: a histidine buffer with pH 6.0 made from 15 mM histidine and 15 mM histidine hydrochloride.

In some embodiments, the above-mentioned histidine buffer is a histidine-acetate buffer; preferably, the molar ratio of histidine to acetate is 1 : 1 to 1.5 : 1; preferably, the pH of such a buffer is 5.5 ± 0.3, preferably about 5.5; preferably, such a buffer contains 15-20 mM histidine and 12-15 mM acetic acid.

In some embodiments, the above-mentioned buffer solution is an acetate buffer; preferably, the acetate buffer is an acetic acid-sodium acetate buffer or an acetic acid-potassium acetate buffer, preferably an acetic acid-sodium acetate buffer. In some embodiments, the acetate buffer is made from 1-30 mM acetic acid and 1-30 mM sodium acetate. In some embodiments, the acetate buffer is made from acetic acid and sodium acetate in a molar ratio of about 1 : 2.1. In some embodiments, the acetate buffer is made from acetic acid and sodium acetate in a molar ratio of about 1 : 5.7. In some embodiments, the acetate buffer is: an acetate buffer with a pH of about 5.0 made from about 6.5 mM acetic acid and about 13.5 mM sodium acetate. In some embodiments, the acetate buffer is: an acetate buffer with a pH of about 5.5 made from about 3 mM acetic acid and about 17 mM sodium acetate.

In some embodiments, the above-mentioned buffer solution is a citrate buffer; preferably, the citrate buffer is a citric acid-sodium citrate buffer.

In some embodiments, the above-mentioned buffer solution has a concentration of about 1-200 mM, preferably about 5-200 mM, preferably about 10-50 mM, preferably about 10-30 mM, preferably about 20-30 mM. Non-limiting examples of the concentration of the above-mentioned buffer solution are about 5 mM, 10 mM, 15 mM, 20 mM, 25 mM, 30 mM, 40 mM, 45 mM, 50 mM, 60 mM, 70 mM, 80 mM, 90 mM, 100 mM, 105 mM, 110 mM, 115 mM, 120 mM, 130 mM, 140 mM, 150 mM, 160 mM, 170 mM or 180 mM or a range formed by any two values within these ranges as endpoints, preferably 10 mM, 15 mM, 20 mM or 30 mM.

In some embodiments, the pH of the above-mentioned buffer solution is about 5.0-6.5, preferably about 5.0-6.0, preferably about 5.5-6.5, preferably about 5.0-5.5, preferably about 5.5-6.0, preferably about 6.0-6.5. Non-limiting examples of the pH of the above-mentioned buffer solution are about 5.0, 5.1, 5.2, 5.3, 5.4, 5.5, 5.6, 5.7, 5.8, 5.9, 6.0, 6.1, 6.2, 6.3, 6.4 and 6.5, preferably about 5.0, 5.5 or 6.0.

In some embodiments, the above-mentioned pharmaceutical composition further comprises a stabilizer selected from one or more of arginine hydrochloride, sodium chloride, mannitol, sorbitol, sucrose and trehalose; preferably, the stabilizer is trehalose or sucrose.

In some embodiments, the above-mentioned stabilizer has a concentration of about 10 mM-400 mM, preferably 20 mM-300 mM, and more preferably 30 mM-200 mM.

In some embodiments, the above-mentioned stabilizer is sodium chloride with a concentration of about 30-200 mM; or the stabilizer is mannitol with a concentration of about 100-300 mM; or the stabilizer is sorbitol with a concentration of about 100-300 mM; or the stabilizer is sucrose with a concentration of about 100-300 mM; or the stabilizer is trehalose with a concentration of about 100-300 mM; or the stabilizer is arginine hydrochloride with a concentration of about 30-200 mM.

In some embodiments, the above-mentioned stabilizer is a combination of about 30-200 mM sodium chloride and about 30-200 mM mannitol; or the stabilizer is a combination of about 30-200 mM sodium chloride and about 30-200 mM trehalose; or the stabilizer is a combination of about 30-200 mM sodium chloride and about 30-200 mM sucrose.

In some embodiments, the above-mentioned stabilizer is sodium chloride. In some embodiments, the above-mentioned stabilizer is sodium chloride with a concentration of about 30-200 mM, and the concentration of the above-mentioned sodium chloride is preferably about 50-190 mM, preferably about 100-180 mM, preferably about 120-170 mM, preferably about 130-150 mM. Non-limiting examples of the concentration of the above-mentioned sodium chloride are about 100 mM, 110 mM, 120 mM, 125 mM, 130 mM, 135 mM, 140 mM, 145 mM, 150 mM, 155 mM, 160 mM, 170 mM, 180 mM, 190 mM and 200 mM, preferably 135 mM or 140 mM.

In some embodiments, the above-mentioned stabilizer is mannitol. In some embodiments, the above-mentioned stabilizer is mannitol with a concentration of about 100-300 mM, and the concentration of the above-mentioned mannitol is preferably about 150-300 mM, preferably about 200-280 mM. Non-limiting examples of the concentration of the above-mentioned mannitol are about 200 mM, 210 mM, 220 mM, 230 mM, 240 mM, 250 mM, 260 mM, 270 mM and 280 mM, preferably 240 mM.

In some embodiments, the above-mentioned stabilizer is sorbitol. In some embodiments, the above-mentioned stabilizer is sorbitol with a concentration of about 100-300 mM, and the concentration of the above-mentioned sorbitol is preferably about 150-300 mM, preferably about 200-280 mM. Non-limiting examples of the concentration of the above-mentioned sorbitol are about 200 mM, 210 mM, 220 mM, 230 mM, 240 mM, 250 mM, 260 mM, 270 mM and 280 mM, preferably 240 mM.

In some embodiments, the above-mentioned stabilizer is sucrose. In some embodiments, the above-mentioned stabilizer is sucrose with a concentration of about 100-300 mM, and the concentration of the above-mentioned sucrose is preferably about 150-300 mM, preferably about 200-280 mM. Non-limiting examples of the concentration of the above-mentioned sucrose are about 200 mM, 210 mM, 220 mM, 230 mM, 240 mM, 250 mM, 260 mM, 270 mM and 280 mM, preferably 220 mM.

In some embodiments, the above-mentioned stabilizer is trehalose. In some embodiments, the above-mentioned stabilizer is trehalose with a concentration of about 100-300 mM, and the concentration of the above-mentioned trehalose is preferably about 150-300 mM, preferably about 200-280 mM. Non-limiting examples of the concentration of the above-mentioned trehalose are about 180 mM, 200 mM, 210 mM, 220 mM, 230 mM, 240 mM, 250 mM, 260 mM, 270 mM and 280 mM, preferably 220 mM.

In some embodiments, the above-mentioned stabilizer is arginine hydrochloride. In some embodiments, the above-mentioned stabilizer is arginine hydrochloride with a concentration of about 30-200 mM, and the concentration of the above-mentioned arginine hydrochloride is preferably about 50-190 mM, preferably about 100-180 mM, preferably about 120-170 mM, preferably about 130-150 mM. Non-limiting examples of the concentration of the above-mentioned arginine hydrochloride are about 100 mM, 110 mM, 120 mM, 125 mM, 130 mM, 135 mM, 140 mM, 145 mM, 150 mM, 155 mM, 160 mM, 170 mM, 180 mM, 190 mM and 200 mM, preferably 135 mM or 140 mM.

In some embodiments, the above-mentioned stabilizer is a combination of sodium chloride and mannitol. In some embodiments, the above-mentioned stabilizer is a combination of about 30-200 mM sodium chloride and about 30-200 mM mannitol, preferably a combination of about 40-150 mM sodium chloride and about 40-180 mM mannitol, preferably a combination of about 40-100 mM sodium chloride and about 80-160 mM mannitol. Non-limiting examples of the above-mentioned stabilizer are a combination of about 50 mM sodium chloride and about 120 mM mannitol, or a combination of about 50 mM sodium chloride and about 140 mM mannitol.

In some embodiments, the above-mentioned stabilizer is a combination of sodium chloride and sucrose. In some embodiments, the above-mentioned stabilizer is a combination of about 30-200 mM sodium chloride and about 30-200 mM sucrose, preferably a combination of about 40-150 mM sodium chloride and about 40-180 mM sucrose, preferably a combination of about 40-100 mM sodium chloride and about 80-160 mM sucrose. Non-limiting examples of the above-mentioned stabilizer are a combination of about 50 mM sodium chloride and about 120 mM sucrose, or a combination of about 50 mM sodium chloride and about 140 mM sucrose.

In some embodiments, the above-mentioned stabilizer is a combination of sodium chloride and trehalose. In some embodiments, the above-mentioned stabilizer is a combination of about 30-200 mM sodium chloride and about 30-200 mM trehalose, preferably a combination of about 40-150 mM sodium chloride and about 40-180 mM trehalose, preferably a combination of about 40-100 mM sodium chloride and about 80-160 mM trehalose. Non-limiting examples of the above-mentioned stabilizer are a combination of about 50 mM sodium chloride and about 120 mM trehalose, or a combination of about 50 mM sodium chloride and about 140 mM trehalose.

In some embodiments, the above-mentioned pharmaceutical composition further comprises a surfactant selected from polysorbate 80, polysorbate 20 or poloxamer 188.

In some embodiments, the above-mentioned surfactant is polysorbate 80.

In some embodiments, the above-mentioned surfactant is polysorbate 20.

In some embodiments, the above-mentioned surfactant has a concentration of about 0.001%-0.1%, preferably about 0.01%-0.1%, preferably about 0.02%-0.08%, on w/v basis. As a non-limiting example, the above-mentioned surfactant has a concentration of about 0.02%, 0.04% or 0.08%, preferably 0.02%.

In some embodiments, the above-mentioned anti-TIGIT antibody or antigen-binding fragment thereof is selected from a murine antibody, a chimeric antibody and a humanized antibody, preferably a humanized antibody.

In some embodiments, the above-mentioned anti-TIGIT antibody or antigen-binding fragment thereof has a heavy chain variable region as represented by SEQ ID NO: 7, and a light chain variable region as represented by SEQ ID NO:8.

In some embodiments, the above-mentioned anti-TIGIT antibody or antigen-binding fragment thereof has a heavy chain variable region as represented by SEQ ID NO: 9, and a light chain variable region as represented by SEQ ID NO:10.

In some embodiments, the above-mentioned anti-TIGIT antibody or antigen-binding fragment thereof has a heavy chain amino acid sequence as represented by SEQ ID NO:11, and a light chain amino acid sequence as represented by SEQ ID NO:12.

In some embodiments, the above-mentioned anti-TIGIT antibody or antigen-binding fragment thereof has a heavy chain amino acid sequence as represented by SEQ ID NO:13, and a light chain amino acid sequence as represented by SEQ ID NO:14.

In some embodiments, the pharmaceutical composition comprises the components as shown in any one of the following (1) - (8), or consists of the components as shown in any one of (1) - (8), respectively:

(1) (a) about 10 mg/mL-80 mg/mL anti-TIGIT antibody or antigen-binding fragment thereof; (b) about 10-30 mM acetate buffer, with a pH of about 5.0; (c) about 100-300 mM sorbitol; and (d) about 0.01%-0.1% polysorbate 80; or

(2) (a) about 10 mg/mL-80 mg/mL anti-TIGIT antibody or antigen-binding fragment thereof; (b) about 10-30 mM acetate buffer, with a pH of about 5.5; (c) about 100-300 mM mannitol; and (d) about 0.01%-0.1% polysorbate 80; or

(3) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM trehalose; and (d) about 0.01%-0.1% polysorbate 80; or

(4) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM sucrose; and (d) about 0.01%-0.1% polysorbate 80; or

(5) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM sucrose; and (d) about 0.01%-0.1% polysorbate 80; or

(6) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM trehalose; and (d) about 0.01%-0.1% polysorbate 80; or

(7) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM trehalose; (d) about 30-200 mM sodium chloride; and (e) about 0.01%-0.1% polysorbate 80; or

(8) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM sucrose; (d) about 30-200 mM sodium chloride; and (e) about 0.01%-0.1% polysorbate 80.

Preferably, the anti-TIGIT antibody is as described in any one of the embodiments herein.

In some embodiments, the pharmaceutical composition comprises the components as shown in any one of the following (1) - (7), or consists of the components as shown in any one of (1) - (7), respectively:

(1) (a) about 40 mg/mL anti-TIGIT antibody or antigen-binding fragment thereof; (b) about 20 mM acetate buffer, with a pH of about 5.0; (c) about 220 mM sucrose; and (d) about 0.02% polysorbate 80; or

(2) (a) about 40 mg/mL anti-TIGIT antibody or antigen-binding fragment thereof; (b) about 20 mM acetate buffer, with a pH of about 5.0; (c) about 220 mM trehalose; and (d) about 0.02% polysorbate 80; or

(3) (a) about 40 mg/mL anti-TIGIT antibody or antigen-binding fragment thereof; (b) about 20 mM acetate buffer, with a pH of about 5.5; (c) about 240 mM mannitol; and (d) about 0.02% polysorbate 80; or

(4) (a) about 40 mg/mL anti-TIGIT antibody or antigen-binding fragment thereof; (b) about 20 mM acetate buffer, with a pH of about 5.5; (c) about 220 mM sucrose; and (d) about 0.02% polysorbate 80; or

(5) (a) about 40 mg/mL anti-TIGIT antibody or antigen-binding fragment thereof; (b) about 20 mM acetate buffer, with a pH of about 5.5; (c) about 220 mM trehalose; and (d) about 0.02% polysorbate 80; or

(6) (a) about 40 mg/mL anti-TIGIT antibody or antigen-binding fragment thereof; (b) about 20 mM acetate buffer, with a pH of about 5.5; (c) about 140 mM trehalose; (d) about 50 mM sodium chloride; and (e) about 0.02% polysorbate 80; or

(7) (a) about 40 mg/mL anti-TIGIT antibody or antigen-binding fragment thereof; (b) about 20 mM acetate buffer, with a pH of about 5.5; (c) about 140 mM sucrose; (d) about 50 mM sodium chloride; and (e) about 0.02% polysorbate 80.

Preferably, the anti-TIGIT antibody is as described in any one of the embodiments herein.

In some embodiments, the pharmaceutical composition is a liquid preparation or a lyophilized preparation.

In some embodiments, the pharmaceutical composition is a liquid preparation.

In some embodiments, the above-mentioned liquid preparation or lyophilized preparation is stable at 2°C-8°C for at least 3 months, at least 6 months, at least 12 months, at least 18 months or at least 24 months.

In some embodiments, the above-mentioned liquid preparation or lyophilized preparation is stable at 40°C for at least 7 days, at least 14 days or at least 28 days.

The present invention further provides the use of the above-mentioned pharmaceutical composition in the preparation of a drug for treating or preventing a TIGIT-mediated disease or condition; preferably, the disease or condition is cancer.

**Brief Description of the Drawings**

FIG. 1: Detection of the effect of humanized antibody on T cell activity by Luciferase assay.

FIG. 2: Detection of the blocking effect of humanized antibodies on the binding of TIGIT to ligand PVR by FACS.

FIG. 3: Pharmacodynamic study of humanized anti-TIGIT antibody and a combination of humanized anti-TIGIT antibody and anti-PD-1 antibody in MC38 tumour-bearing TIGIT transgenic mice.

## Detailed Description of Embodiments

### Definition and Description

In order that the present invention may be more readily understood, certain technical and scientific terms are specifically defined below. Unless explicitly defined otherwise herein, all other technical and scientific terms used herein have the same meaning as commonly understood by one of ordinary skill in the art to which the present invention pertains. It is to be understood that the present invention is not limited to particular methods, reagents, compounds, compositions or biological systems, which can, of course, vary. It is also to be understood that the terminology used in the present application is for the purpose of describing particular embodiments only and is not intended to be limiting.

Unless the content is clearly stated otherwise, the singular forms "a", "an" and "the" used in the description and the appended claims include plural referents. Therefore, for example, reference to "a polypeptide" includes a combination of two or more polypeptides.

The term "pharmaceutical composition" or "preparation" denotes a mixture containing one or more of the antibodies described herein and other components, such as a physiologically and pharmaceutically acceptable carrier and an excipient. The purpose of a pharmaceutical composition is to promote the administration to an organism, which will facilitate the absorption of active ingredients and thus exert biological activities.

The term "liquid preparation" refers to a preparation in a liquid state and is not intended to refer to a resuspended lyophilized preparation. The liquid preparation of the present invention is stable on storage, and the stability thereof is independent of lyophilization (or other state-changing methods, such as spray drying).

The term "aqueous liquid preparation" refers to a liquid preparation using water as a solvent. In some embodiments, the aqueous liquid preparation is a preparation that does not require lyophilization, spray drying and/or freezing to maintain stability (e.g., chemical and/or physical stability and/or biological activity).

The term "excipient" refers to a reagent that can be added to a preparation to provide desired properties (e.g., consistency and improved stability) and/or to adjust osmotic pressure. Examples of common excipients include, but are not limited to, sugars, polyols, amino acids, surfactants, and polymers.

As used in the present application, "about" when referring to a measurable value (e.g., amount, duration, etc.) is intended to encompass a variation of $\pm$ 20% or $\pm$ 10% from the specified value, including $\pm$ 5%, $\pm$ 1% and $\pm$ 0.1% as these variations are suitable for carrying out the disclosed method.

The term "buffer with a pH of about 5.0-6.5" refers to a reagent which renders a solution comprising the reagent resistant to pH changes through the action of its acid/base conjugate component. The buffer used in the preparation of the present invention may have a pH in the range of about 5.0 to about 6.5, or a pH in the range of about 5.5 to about 6.5, or a pH in the range of about 5.0 to about 6.0.

Herein, examples of "buffer" that controls pH within this range include acetate (e.g., sodium acetate), succinate (e.g., sodium succinate), gluconic acid, histidine, histidine hydrochloride, methionine, citrate, phosphate, citrate/phosphate, imidazole, acetic acid, acetate and a combination thereof, and other organic acid buffers.

A "histidine buffer" is a buffer comprising histidine ions. Examples of the histidine buffer include histidine and salts of histidine, such as histidine hydrochloride, histidine acetate, histidine phosphate and histidine sulphate, such as a histidine buffer containing histidine and histidine hydrochloride. The histidine buffer of the present invention also includes a histidine buffer containing histidine and acetate (e.g., sodium acetate or potassium acetate).

A "citrate buffer" is a buffer comprising citrate ions. Examples of the citrate buffer include citric acid-sodium citrate, citric acid-potassium citrate, citric acid-calcium citrate, citric acid-magnesium citrate, etc. The preferred citrate buffer is a citric acid-sodium citrate buffer.

An "acetate buffer" is a buffer comprising acetate ions. Examples of the acetate buffer include acetic acid-sodium acetate, acetic acid-potassium acetate, acetic acid-calcium acetate, acetic acid-magnesium acetate, etc. The preferred acetate buffer is an acetic acid-sodium acetate buffer.

A "succinate buffer" is a buffer comprising succinate ions. Examples of the succinate buffer include succinic acid-sodium succinate, succinic acid-potassium succinate, succinic acid-calcium succinate, succinic acid-magnesium succinate, etc. The preferred succinate buffer is a succinic acid-sodium succinate buffer.

The term "stabilizer" denotes a pharmaceutically acceptable excipient which protects active pharmaceutical ingredients and/or preparations from chemical and/or physical degradation during manufacture, storage and application. The stabilizers include, but are not limited to, sugars, amino acids, salts, polyols and their metabolites as defined below, e.g., sodium chloride, calcium chloride, magnesium chloride, mannitol, sorbitol, sucrose, trehalose, arginine or salts thereof (such as arginine hydrochloride), glycine, alanine ($\alpha$-alanine and $\beta$-alanine), betaine, leucine, lysine, glutamic acid, aspartic acid, proline, 4-hydroxyproline, sarcosine, $\gamma$-aminobutyric acid (GABA), opines, alanopine, octopine, strombine, trimethylamine N-oxide (TMAO), human serum albumin (HSA), bovine serum albumin (BSA), $\alpha$-casein, globulin, $\alpha$-lactalbumin, LDH, lysozyme, myoglobin, ovalbumin, and RNAaseA. Some stabilizers, such as sodium chloride, calcium chloride, magnesium chloride, mannitol, sorbitol and sucrose, can also play a role in controlling osmotic pressure. The stabilizer specifically used in the present invention is selected from one or more of polyols, amino acids, salts and sugars. The preferred salt is sodium chloride; the preferred sugars are sucrose and trehalose; and the preferred polyols are sorbitol and mannitol. The preferred amino acids are arginine or salts thereof (such as arginine hydrochloride), glycine and proline. The preferred stabilizers are sodium chloride, mannitol, sorbitol, sucrose, trehalose, arginine hydrochloride, glycine, proline, sodium chloride-sorbitol, sodium chloride-mannitol, sodium chloride-sucrose, sodium chloride-trehalose, arginine hydrochloride-mannitol, and arginine hydrochloride-sucrose.

The term "surfactant" generally includes a reagent that protects a protein, e.g., an antibody, from air/solution interface induced stress and solution/surface induced stress to reduce aggregation of the antibody or to minimize the formation of particulate matter in a preparation. Exemplary surfactants include, but are not limited to, nonionic surfactants, e.g., polyoxyethylene sorbitan fatty acid esters (such as polysorbate 20 and polysorbate 80), polyethylene-polypropylene copolymers, polyethylene-polypropylene glycol, polyoxyethylene-stearate, polyoxyethylene alkyl ethers (such as polyoxyethylene monolauryl ether), alkyl phenyl polyoxyethylene ether (Triton-X), polyoxyethylene-polyoxypropylene copolymers (poloxamer, Pluronic) and sodium dodecyl sulphate (SDS).

The term "isotonicity" means that the preparation has substantially the same osmotic pressure as human blood. An isotonic preparation generally has an osmotic pressure of about 250 to 350 mOsm. Isotonicity can be measured using a vapor pressure or freezing point depression osmometer.

The term "stable" preparation refers to a preparation in which an antibody substantially retains its physical and/or chemical stability and/or biological activity during the manufacturing process and/or storage. A pharmaceutical preparation may be stable even if the contained antibody fails to retain 100% of its chemical structure or biological function after a certain period of storage. In some cases, an antibody that may retain about 90%, about 95%, about 96%, about 97%, about 98% or about 99% of its structure or function after a certain period of storage may also be considered "stable". Various analytical techniques for measuring protein stability are available in the art and are reviewed in Peptide and Protein Drug Delivery 247-301, edited by Vincent Lee, Marcel Dekker, Inc., New York, N.Y., Pubs. (1991), and Jones, A. (1993) Adv. Drug Delivery Rev. 10: 29-90 (both incorporated by reference).

The stability of a preparation can be measured by, among other methods, determining the percentage of native antibodies remaining in the preparation after storage at a certain temperature for a certain period of time. Among other methods, the percentage of native antibodies can be measured by size exclusion chromatography (e.g., size exclusion chromatography-high performance liquid chromatography [SEC-HPLC]), with "native" referring to unaggregated and undegraded. In some embodiments, protein stability is determined as the percentage of monomeric proteins in a solution with a low percentage of degraded (e.g., fragmented) and/or aggregated proteins. In some embodiments, the preparation can be stable for storage at room temperature, about 25°C-30°C or 40°C for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer, with no more than about 6%, 5%, 4%, 3%, 2%, 1%, 0.5% or 0.1% of antibodies in an aggregated form.

Stability can be measured by, among other methods, determining the percentage of an antibody ("acidic form") migrating in the more acidic fraction of this antibody main fraction ("main charge form") during ion exchange, where the stability is inversely proportional to the percentage of the antibody in an acidic form. Among other methods, the percentage of "acidified" antibody can be measured by ion exchange chromatography (e.g., cation exchange high performance liquid chromatography [CEX-HPLC]). In some embodiments, an acceptable degree of stability means that upon storage of the preparation at a certain temperature for a certain period of time, the detectable antibodies in an acidic form are no more than about 49%, 45%, 40%, 35%, 30%, 25%, 20%, 15%, 10%, 5%, 4%, 3%, 2%, 1%, 0.5% or 0.1%. The certain period of time of storage prior to measuring stability can be at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer. When assessing stability, a certain temperature that allows storage of a pharmaceutical preparation can be any temperature in a range of about -80°C to about 45°C, for example, storage at about -80°C, about -30°C, about -20°C, about 0°C, about 2°C-8°C, about 5°C, about 25°C or about 40°C.

An antibody "retains its physical stability" in the pharmaceutical composition if the antibody shows substantially no evidence of, for example, aggregation, precipitation and/or denaturation upon visual inspection of colour and/or clarity or as measured by UV light scattering or by size exclusion chromatography. Aggregation is a process by which individual molecules or complexes associate covalently or non-covalently to form aggregates. Aggregation can proceed to the degree where a visible precipitate is formed.

The stability of a preparation, for example, physical stability, can be assessed by methods well known in the art, including measuring the apparent extinction (absorbance or optical density) of a sample. Such an extinction measurement correlates with the turbidity of a preparation. The turbidity of a preparation is, in part, an inherent property of proteins dissolved in a solution, and is typically measured by turbidimetry, and is measured in nephelometric turbidity unit (NTU).

A turbidity level that varies with, for example, the concentration of one or more components (e.g., protein and/or salt concentration) in a solution is also referred to as the "opacification" or "opaque appearance" of a preparation. A turbidity level can be calculated with reference to a standard curve generated using a suspension having known turbidity. Reference standards for determining the turbidity level of a pharmaceutical composition can be based on the European Pharmacopoeia standard (European Pharmacopoeia, 4th edition, "Directorate for the Quality of Medicine of the Council of Europe" (EDQM), Strasbourg, France). According to the European Pharmacopoeia standard, a clear solution is defined as a solution having a turbidity lower than or equal to that of a reference suspension having a turbidity of about 3. Turbidity measurement in turbidimetry can detect Rayleigh scattering, which typically varies linearly with concentration, in the absence of association or non-ideal effects. Other methods for assessing physical stability are well known in the art.

An antibody "retains its chemical stability" in a pharmaceutical composition if its chemical stability at a given time point is such that the antibody is considered to still retain its biological activity as defined hereinafter. Chemical stability may be assessed by, for example, detecting or quantifying the chemically changed form of an antibody. Chemical changes may include size changes (e.g., clipping), which can be assessed using, for example, size exclusion chromatography, SDS-PAGE, and/or matrix-assisted laser desorption ionization/time-of-flight mass spectrometry (MALDI/TOF MS). Other types of chemical changes include charge changes (for example, resulting from deamidation or oxidation), which can be assessed by, for example, ion exchange chromatography.

An antibody "retains its biological activity" in a pharmaceutical composition if the antibody in the pharmaceutical composition is biologically active for its intended purpose. For example, if after a preparation is stored at a temperature, such as 5°C, 25°C and 45°C, for a certain period of time (e.g., 1 to 12 months), the binding affinity of the anti-TIGIT antibody contained in the preparation to TIGIT is at least 90%, 95% or more of the binding affinity of the antibody prior to the storage, then the preparation of the present invention is considered stable. Binding affinity may also be determined using techniques such as ELISA or plasmon resonance.

In the context of the present invention, a "therapeutically effective amount" or "effective amount" of an antibody in the pharmacological sense refers to an amount effective in the prevention or treatment or alleviation of the symptoms of the disorder that the antibody can effectively treat. In the present invention, a "therapeutically effective amount" or "therapeutically effective dose" of a drug is any amount of a drug that protects a subject from the onset of a disease or promotes the regression of a disease when used alone or in combination with another therapeutic agent. The regression of the disease is evidenced by the reduction in the severity of the symptoms of the disease, the increase in the frequency and duration of the asymptomatic period of the disease, or the prevention of injury or disability caused by the pain of the disease. The ability of a drug to promote disease regression can be evaluated using a variety of methods known to a person skilled in the art, such as determining the activity of the agent in human subjects during clinical trials, in animal model systems that predict the efficacy in human, or by in vitro assays. A therapeutically effective amount of a drug includes a "prophylactically effective amount", that is, any amount of a drug that inhibits the development or recurrence of a disease when administered to a subject at risk of developing the disease or a subject suffering from recurrence of the disease, alone or in combination with other therapeutic drugs.

The term "subject" or "patient" is intended to include mammalian organisms. Examples of subjects/patients include human and non-human mammals, such as non-human primates, dogs, cows, horses, pigs, sheep, goats, cats, mice, rabbits, rats and transgenic non-human animals. In a specific embodiment of the present invention, the subject is human.

The terms "apply", "administer" and "treat" refer to the introduction of a composition comprising a therapeutic agent into a subject using any one of various methods or delivery systems known to a person skilled in the art. The administration route of an anti-PD-1 antibody includes intravenous, intramuscular, subcutaneous, peritoneal, spinal or other parenteral administration routes, such as injection or infusion. "Parenteral administration" refers to administration routes usually by injection other than enteral or local administration, including but not limited to intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, intradural and intrasternal injection and infusion, and in vivo electroporation.

Anti-TIGIT antibody

The term "antibody" as used herein should be understood to include an intact antibody molecule and an antigen-binding fragment thereof. The term "antigen-binding moiety" or "antigen-binding fragment" (or simply "antibody moiety" or "antibody fragment") of an antibody as used herein refers to one or more fragments in the antibody that retain the ability to specifically bind to human TIGIT or an epitope thereof.

The term "full-length antibody" or "intact antibody molecule" as used herein refers to an immunoglobulin molecule comprising four peptide chains, two heavy (H) chains (about 50-70 kDa in full length) and two light (L) chains (about 25 kDa in full length) interconnected by disulphide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further subdivided into highly variable complementarity determining regions (CDRs) and more conserved regions called framework regions (FRs) spaced by CDRs. Each VH or VL region consists of 3 CDRs and 4 FRs arranged from amino terminal to carboxyl terminal, in the following order: FR1, CDR1, FR2, CDR2, FR3, CDR3, FR4. The variable regions of the heavy and light chains contain binding domains that interact with antigens. The constant region of the antibody can mediate the binding of the immunoglobulin to a host tissue or factor (comprising various cells (e.g., effector cells) of the immune system and the first component of the classical complement system (Clq)).

As used herein, the term "CDR" refers to a complementarity determining region within the variable sequence of an antibody. There are 3 CDRs in each variable region of the heavy and light chains, and the CDRs are designated HCDR1, HCDR2 and HCDR3 or LCDR1, LCDR2 and LCDR3 for each heavy and light chain variable region. The exact boundaries of these CDRs are defined differently according to different systems.

The precise amino acid sequence boundaries of the variable region CDRs of the antibody of the present invention can be determined using any of a number of well-known schemes, including Chothia based on the three-dimensional structure of an antibody and the topology of a CDR loop (Chothia et al., (1989) Nature 342: 877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (1999 Nucleic Acids Research, 27, 209-212), and the North CDR definition based on affinity propagation clustering using a large number of crystal structures. The boundaries of the CDRs of the antibody of the present invention can be determined by a person skilled in the art according to any scheme in the art (e.g., different assignment systems or combinations).

As used herein, "antigen-binding fragment" includes a fragment of an antibody or a derivative thereof, typically including at least a fragment of the antigen-binding region or the variable region (e.g., one or more CDRs) of the parent antibody, and the fragment of the antibody or the derivative thereof retains at least some of the binding specificity of the parent antibody. Examples of the antigen-binding fragment include, but are not limited to, Fab, Fab', F(ab')2 and Fv fragments; diabodies; linear antibodies; single-stranded antibody molecules, such as sc-Fv; and nanobodies and multispecific antibodies formed from antibody fragments. When the binding activity of an antibody is expressed on a molar concentration basis, a binding fragment or a derivative thereof typically retains at least 10% of the antigen binding activity of the parent antibody. Preferably, the binding fragment or the derivative thereof retains at least 20%, 50%, 70%, 80%, 90%, 95% or 100% or more of the antigen binding affinity of the parent antibody. It is also contemplated that an antigen binding fragment of an antibody may include conservative or non-conservative amino acid substitutions that do not significantly change its biological activity (referred to as "conservative variants" or "functionally conservative variants" of the antibody).

The anti-TIGIT antibody or the antigen-binding fragment thereof of the present invention includes any one of the anti-TIGIT antibodies or the antigen-binding fragments thereof described in the application number PCT/CN 2020/101883, the entire content of which is incorporated herein by reference. In some embodiments, the CDR sequences of the antibodies used in the methods and compositions of the present invention include the CDR sequences from the antibody hu20 or hu3 described in PCT/CN 2020/101883.

The non-limiting, exemplary antibodies used in the examples herein are selected from the humanized antibodies hu20 and hu3 described in PCT/CN 2020/101883, and the CDR amino acid sequences, variable region sequences and full-length amino acid sequences of these humanized antibodies are as shown in Table 1.

Table 1: Full-length, variable region and CDR amino acid sequences of humanized antibodies (IMGT definition

| Antibody | hu20 | hu3 |
|---|---|---|
| HCDR1 | SEQ ID NO:1 | SEQ ID NO: 1 |
| HCDR2 | SEQ ID NO:2 | SEQ ID NO:2 |
| HCDR3 | SEQ ID NO:3 | SEQ ID NO:3 |
| LCDR1 | SEQ ID NO:4 | SEQ ID NO:4 |
| LCDR2 | SEQ ID NO:5 | SEQ ID NO:5 |
| LCDR3 | SEQ ID NO:6 | SEQ ID NO:6 |
| VH | SEQ ID NO:7 | SEQ ID NO:9 |
| VL | SEQ ID NO:8 | SEQ ID NO:10 |

(continued)

| Antibody | hu20 | hu3 |
|---|---|---|
| HC | SEQ ID NO:11 | SEQ ID NO:13 |
| LC | SEQ ID NO:12 | SEQ ID NO:14 |

Pharmaceutical preparation

The pharmaceutical composition of the present invention is a highly stable pharmaceutical composition comprising a humanized antibody that specifically binds to TIGIT. The present invention finds that trehalose can obviously improve the stability of the pharmaceutical composition.

The pharmaceutical composition of the present invention is a liquid preparation having high stability. In particular, the present invention finds that preparations containing an acetate buffer are significantly more stable than preparations containing other buffers.

The present invention provides a pharmaceutical composition, comprising: (1) a buffer solution; and (2) an anti-TIGIT antibody or an antigen-binding fragment thereof.

The antibody in the pharmaceutical composition of the present invention can be a murine antibody, a chimeric antibody, and a humanized antibody, preferably a humanized antibody, and can have HCDR1, HCDR2 and HCDR3 as respectively represented by SEQ ID NO: 1, SEQ ID NO.2, and SEQ ID NO:3, and LCDR1, LCDR2 and LCDR3 as respectively represented by SEQ ID NO:4, SEQ ID NO:5, and SEQ ID NO: 6. Preferably, the antibody in the pharmaceutical composition of the present invention has a heavy chain variable region as represented by SEQ ID NO:7 and a light chain variable region as represented by SEQ ID NO:8, or has a heavy chain variable region as represented by SEQ ID NO:9 and a light chain variable region as represented by SEQ ID NO: 10. More preferably, the antibody in the pharmaceutical composition of the present invention has a heavy chain amino acid sequence as represented by SEQ ID NO: 11 and a light chain amino acid sequence as represented by SEQ ID NO: 12, respectively, or the antibody in the pharmaceutical composition of the present invention has a heavy chain amino acid sequence as represented by SEQ ID NO: 13 and a light chain amino acid sequence as represented by SEQ ID NO: 14, respectively.

The anti-TIGIT antibody or the antigen-binding fragment thereof in the pharmaceutical composition of the present invention has a concentration of about 1-300 mg/mL, preferably about 1-250 mg/mL, preferably about 1-200 mg/mL, preferably about 10-80 mg/mL, and more preferably 10-40 mg/mL. More preferably, the above-mentioned anti-TIGIT antibody or antigen-binding fragment thereof has a concentration of about 5 mg/mL, 10 mg/mL, 15 mg/mL, 20 mg/mL, 25 mg/mL, 30 mg/mL, 35 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL or 140 mg/mL, preferably about 10 mg/mL, 20 mg/mL or 40 mg/mL.

The buffer in the pharmaceutical composition of the present invention can be selected from an acetate buffer, a citrate buffer and a histidine buffer to provide a pH of 5.0 to 6.5, preferably 5.0 to 6.0, more preferably 5.5 ± 0.3, and more preferably about 5.5 for the pharmaceutical composition of the present invention. On the other hand, the pH of the buffer used in the pharmaceutical composition of the present invention may be 5.0-6.5, preferably 5.0-6.0, more preferably 5.5 ± 0.3, and more preferably about 5.5.

The particularly preferred buffer in the pharmaceutical composition of the present invention is an acetate buffer. Preferably, the pH of the acetate buffer is about 5.0 to about 6.0, more preferably about 5.5 ± 0.3. Preferably, the acetate buffer is an acetic acid-sodium acetate buffer or an acetic acid-potassium acetate buffer, preferably an acetic acid-sodium acetate buffer. In some embodiments, the acetate buffer is made from 1-30 mM acetic acid and 1-30 mM sodium acetate. In some embodiments, the acetate buffer is made from acetic acid and sodium acetate in a molar ratio of about 1 : 2.1. In some embodiments, the acetate buffer is made from acetic acid and sodium acetate in a molar ratio of about 1 : 5.7. In some embodiments, the acetate buffer is: an acetate buffer with a pH of about 5.0 made from about 6.5 mM acetic acid and about 13.5 mM sodium acetate. In some embodiments, the acetate buffer is: an acetate buffer with a pH of about 5.5 made from about 3 mM acetic acid and about 17 mM sodium acetate.

In some other preferred embodiments, the buffer in the pharmaceutical composition of the present invention is a citrate buffer; preferably, the citrate buffer is a citric acid-sodium citrate buffer. Preferably, the pH of the citrate buffer in the pharmaceutical composition of the present invention is about 5.0 to about 6.0.

The buffer in the pharmaceutical composition of the present invention can be a histidine buffer, including a histidine-hydrochloride buffer or a histidine-acetate buffer, preferably a histidine-hydrochloride buffer. More preferably, the histidine-hydrochloride buffer is made from histidine and histidine hydrochloride, preferably L-histidine and L-histidine monohydrochloride. In some embodiments, the histidine buffer is made from 1-20 mM L-histidine and 1-20 mM L-histidine monohydrochloride. In some embodiments, the histidine buffer is made from histidine and histidine hydrochloride in a molar ratio of 1 : 1 to 1 : 4. In some embodiments, the histidine buffer is made from histidine and histidine hydrochloride in a molar ratio of 1 : 1. In some embodiments, the histidine buffer is made from histidine and histidine hydrochloride in a molar ratio of 1 : 3.

Therefore, in the pharmaceutical composition of the present invention, the buffer can be an acetate buffer with pH 5.0-6.0, and has a concentration of 10-30 mM in the pharmaceutical composition; and the pharmaceutical composition contains 10-80 mg/mL anti-TIGIT antibody or antigen-binding fragment thereof according to any one of the preceding embodiments, especially the antibodies hu3 and hu20 or the antigen-binding fragments thereof as described herein.

In some embodiments, the pharmaceutical composition of the present invention further contains a stabilizer. Preferably, the stabilizer is selected from one or more of arginine hydrochloride, sodium chloride, mannitol, sorbitol, sucrose and trehalose. Preferably, the stabilizer is trehalose or sucrose. The stabilizer in the pharmaceutical composition of the present invention has a concentration of about 10 mM-400 mM, preferably 20 mM-300 mM, and more preferably 30 mM-200 mM. In some embodiments, the stabilizer is sodium chloride with a concentration of about 30-200 mM; or the stabilizer is mannitol with a concentration of about 100-300 mM, preferably 200-300 mM; or the stabilizer is sorbitol with a concentration of about 100-300 mM, preferably 200-300 mM; or the stabilizer is sucrose with a concentration of about 100-300 mM, preferably 200-300 mM; or the stabilizer is trehalose with a concentration of about 100-300 mM, preferably 200-300 mM; or the stabilizer is arginine hydrochloride with a concentration of about 30-200 mM, preferably about 60-150 mM. In some embodiments, the stabilizer is a combination of about 30-200 mM sodium chloride and about 30-200 mM mannitol; or the stabilizer is a combination of about 30-200 mM sodium chloride and about 30-200 mM sucrose; or the stabilizer is a combination of about 30-200 mM sodium chloride and about 30-200 mM trehalose.

Thus, in some embodiments, the pharmaceutical composition of the present invention contains: (a) 10-30 mM buffer solution, which is an acetate buffer with pH 5.0-6.0; (b) 10-80 mg/mL anti-TIGIT antibody or antigen-binding fragment thereof according to any one of the preceding embodiments, especially the antibody hu3 or hu20 or the antigen-binding fragment thereof as described herein; and (c) 20 mM-300 mM stabilizer, preferably sodium chloride with a concentration of about 30-200 mM, or mannitol with a concentration of about 200-300 mM, or sorbitol with a concentration of about 200-300 mM, or sucrose with a concentration of about 200-300 mM, or trehalose with a concentration of about 200-300 mM, or arginine hydrochloride with a concentration of about 60-150 mM. In some embodiments, the stabilizer is about 200-300 mM sucrose. In some embodiments, the stabilizer is about 200-300 mM trehalose.

In some embodiments, the pharmaceutical composition of the present invention further includes a surfactant. The preferred surfactant is selected from polysorbate 80, polysorbate 20 and poloxamer 188. The most preferred surfactant is polysorbate 80. The surfactant in the pharmaceutical composition of the present invention has a concentration of about 0.001%-0.1%, preferably about 0.02%-0.08%, preferably about 0.02%-0.04%, on a w/v basis. As a non-limiting example, the surfactant in the pharmaceutical composition of the present invention has a concentration of about 0.02%, 0.04% or 0.08%, preferably 0.02%.

Thus, in some embodiments, the pharmaceutical composition of the present invention contains: a buffer solution, which is an acetic acid-sodium acetate buffer with pH 5.0-6.0 and has a concentration of 10-30 mM in the pharmaceutical composition; 10-80 mg/mL anti-TIGIT antibody or antigen-binding fragment thereof according to any one of the preceding embodiments, especially the antibodies hu3 and hu20 or the antigen-binding fragments thereof as described herein; 100 mM-300 mM stabilizer, preferably, the stabilizer is about 200-300 mM sucrose, or about 200-300 mM trehalose; and 0.02%-0.04% polysorbate 80 on a w/v basis.

The pharmaceutical composition of the present invention can be a liquid preparation or a lyophilized preparation.

Medicinal uses and methods

The present invention further provides the pharmaceutical composition according to any one of the embodiments of the present invention for the treatment or prevention of TIGIT-mediated diseases, the use of the pharmaceutical composition according to any one of the embodiments of the present invention in the preparation of a drug for the treatment or prevention of TIGIT-mediated diseases, and a method for treating or preventing TIGIT-mediated diseases, comprising administering a therapeutically effective amount of the pharmaceutical composition according to any one of the embodiments of the present invention to an individual or patient in need thereof.

In the present invention, TIGIT-mediated diseases refer to diseases in which TIGIT is involved in the occurrence and development of the diseases, including but not limited to cancer.

"Cancer" and "cancerous" refer to or describe the physiological illness in mammals that is often characterised by unregulated cell growth. Included in this definition are benign and malignant cancers as well as dormant tumours or micrometastases. Examples of cancers include, but are not limited to, carcinoma, lymphoma, blastoma, sarcoma, and leukaemia. More specific examples of such cancers include squamous cell carcinoma, lung cancer (including small cell lung cancer, non-small cell lung cancer, adenocarcinoma of the lung, and squamous cell carcinoma of the lung), peritoneal carcinoma, hepatocellular carcinoma, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland cancer, renal cancer or cancer of the kidney, nasopharyngeal carcinoma, oesophageal squamous cell carcinoma, hepatic cancer, prostate cancer, vulvar cancer, thyroid cancer, cancer of the liver, and various types of head and neck cancers, and B-cell lymphoma (including low-grade/follicular non-Hodgkin lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphoma, and Waldenstrom's macroglobulinemia), chronic lymphocytic leukaemia (CLL), acute lymphoblastic leukaemia (ALL), hairy cell leukaemia, chronic myeloblastic leukaemia, and post-transplant lymphoproliferative disorder (PTLD), as well as abnormal vascular proliferation associated with phakomatoses, oedema (such as those associated with brain tumours) and Meigs syndrome.

The present invention will hereinafter be illustrated by way of specific examples. It should be understood that these examples are illustrative only and are not intended to limit the scope of the present invention. The methods and materials used in the examples, unless otherwise stated, are conventional methods and materials in the art.

Example 1: Experiments for screening buffer solutions, pH and protein concentrations

In a liquid pharmaceutical composition, the buffer solution and the pH closely affect the stability of antibodies, and each antibody with unique physicochemical properties has the most suitable buffer type and pH. This example aims to screen an optimal buffer solution and pH, so that the anti-TIGIT antibody disclosed in the present invention has the best stability for clinical application.

This example was performed with antibody hu20 at concentrations of about 10 mg/mL, 20 mg/mL and 40 mg/mL. Samples were subjected to ultrafiltration, concentration and liquid exchange using Millipore Pellicon3 0.11 $m^2$ membrane. After liquid exchange, the samples were in corresponding preparations. The samples were placed in sealed centrifuge tubes for screening buffer solutions. Acetate buffer, citrate buffer and histidine buffer, with a pH ranging from 5.0 to 6.5, were screened (as shown in Table 2). The samples were placed in an environment of 40°C ± 2°C, and were taken out at week 0, week 1, week 2 and week 4, respectively, for appearance and visible particulate detection, and analysis and detection by SEC-HPLC and CEX-HPLC. The main pathway for protein degradation is the formation of aggregates, cleavage products and charged variants. Size exclusion chromatography (SEC-HPLC) was used to determine the percentages of a native form (a protein monomer) and an aggregated form, respectively, and cation exchange chromatography (CEX-HPLC) was used to determine the percentages of antibody in an acidic form and antibody in a basic form, respectively. The monomer content determined by SEC-HPLC and the main peak content determined by CEX-HPLC after the preparations were placed for four weeks (4 W) were used to fit a straight line and the slope of decline (%/week) was calculated to examine the effects of different buffer solutions and pH on the stability of antibody hu20. See Table 3 for the summarized results.

Table 2: Information of preparations in experiments for screening buffer solutions and pH

| Preparation number | pH | Buffer solution | Stabilizer 1 | Stabilizer 2 | Polysorbate 80 | Protein concentration |
|---|---|---|---|---|---|---|
| 1-1 | 5.0 | 20 mM acetate buffer (glacial acetic acid-sodium acetate) | 50 mM sodium chloride | 140 mM mannitol | 0.02% | 20 mg/mL |
| 1-2-1 | 5.5 | 20 mM acetate buffer (glacial acetic acid-sodium acetate) | 50 mM sodium chloride | 140 mM mannitol | 0.02% | 10 mg/mL |
| 1-2-2 | 5.5 | 20 mM acetate buffer (glacial acetic acid-sodium acetate) | 50 mM sodium chloride | 140 mM mannitol | 0.02% | 20 mg/mL |
| 1-2-3 | 5.5 | 20 mM acetate buffer (glacial acetic acid-sodium acetate) | 50 mM sodium chloride | 140 mM mannitol | 0.02% | 40 mg/mL |
| 1-3 | 6.0 | 20 mM citrate buffer (citric acid-sodium citrate) | 50 mM sodium chloride | 140 mM mannitol | 0.02% | 20 mg/mL |
| 1-4 | 6.5 | 20 mM citrate buffer (citric acid-sodium citrate) | 50 mM sodium chloride | 140 mM mannitol | 0.02% | 20 mg/mL |

(continued)

| Preparation number | pH | Buffer solution | Stabilizer 1 | Stabilizer 2 | Polysorbate 80 | Protein concentration |
|---|---|---|---|---|---|---|
| 1-5 | 5.5 | 20 mM histidine buffer (L-histidine -L-histidine hydrochloride) | 50 mM sodium chloride | 140 mM mannitol | 0.02% | 20 mg/mL |

Table 3: Decline rate of monomer content and main peak content under different protein concentrations, pH and buffer solutions

| Preparation number | SEC-HPLC Monomer content decline rate (%/ week) | CEX-HPLC Main peak content decline rate (%/ week) |
|---|---|---|
| 1-1 | 0.6 | 3.0 |
| 1-2-1 | 0.2 | 2.8 |
| 1-2-2 | 0.2 | 2.7 |
| 1-2-3 | 0.3 | 2.8 |
| 1-3 | 0.7 | 3.8 |
| 1-4 | 0.3* | 3.6* |
| 1-5 | 1.3 | 5.0 |
| Notes: * denotes the average of two-week data. | | |

The results of appearance and visible particulates showed that under the accelerated condition of 40°C ± 2°C, a precipitate appeared in preparation 1-4 and the screening experiment was terminated, and other preparation samples were normal, indicating that the citrate buffer solution with pH 6.5 was not suitable as the buffer solution of the present project.

The results of the decline rates determined by SEC-HPLC and CEX-HPLC showed that, after the preparations were placed for 4 weeks at 40°C ± 2°C, the monomer purity and the main peak content declined fastest in the histidine buffer solution with pH 5.5 (preparation 1-5), followed by in the citrate buffer solution with pH 6.0 (preparation 1-3). In the acetate buffer solution with pH 5.5 (preparation 1-2), the purity decline and the main peak content decline were both the lowest under different protein concentrations, with an average purity decline rate of 0.23%/week and an average main peak content decline rate of 2.76%/week. Meanwhile, the protein concentration had little effect on the purity.

Example 2: Experiments for screening stabilizers (excipients)

To further investigate the effect of different excipients on the stability of antibodies, one of the excipients, namely sodium chloride, sucrose, arginine hydrochloride, trehalose, sorbitol or mannitol, was selected for comparative testing. The effects of the above-mentioned different excipients on the stability of monoclonal antibody hu20 with a concentration of 20 mg/ml were examined under the conditions of 20 mM acetate buffer solution with pH 5.0, 20 mM acetate buffer solution with pH 5.5, and 20 mM citrate buffer solution with pH 6.0. The information of specific preparations is as shown in Table 4. Each preparation was placed at 40°C ± 2°C after being subpackaged, and was taken out at week 0, week 1, week 2 and week 4, respectively, for analysis and detection. The change in the monomer content of antibody hu20 was detected by size exclusion chromatography-high performance liquid chromatography (SEC-HPLC), and the main charge peak content of antibody hu20 was detected by weak cation exchange high performance liquid chromatography (CEX-HPLC). Moreover, the appearance and visible particulates were detected. The results are as shown in Table 5.

Table 4: Information of preparations in experiments for screening excipients

| Preparation number | Buffer solution | pH | Excipient | Polysorbate-80 |
|---|---|---|---|---|
| 2-1-1 | 20 mM acetate buffer (glacial acetic acid-sodium acetate) | 5.0 | 135 mM sodium chloride | 0.02% |
| 2-1-2 | | | 135 mM arginine hydrochloride | 0.02% |
| 2-1-3 | | | 240 mM mannitol | 0.02% |
| 2-1-4 | | | 240 mM sorbitol | 0.02% |
| 2-1-5 | | | 220 mM sucrose | 0.02% |
| 2-1-6 | | | 220 mM trehalose | 0.02% |
| 2-2-1 | 20 mM acetate buffer (glacial acetic acid-sodium acetate) | 5.5 | 135 mM sodium chloride | 0.02% |
| 2-2-2 | | | 135 mM arginine hydrochloride | 0.02% |
| 2-2-3 | | | 240 mM mannitol | 0.02% |
| 2-2-4 | | | 240 mM sorbitol | 0.02% |
| 2-2-5 | | | 220 mM sucrose | 0.02% |
| 2-2-6 | | | 220 mM trehalose | 0.02% |
| 2-3-1 | 20 mM citrate buffer (citric acid-sodium citrate) | 6.0 | 135 mM sodium chloride | 0.02% |
| 2-3-2 | | | 135 mM arginine hydrochloride | 0.02% |
| 2-3-3 | | | 240 mM mannitol | 0.02% |
| 2-3-4 | | | 240 mM sorbitol | 0.02% |
| 2-3-5 | | | 220 mM sucrose | 0.02% |
| 2-3-6 | | | 220 mM trehalose | 0.02% |

Table 5: Decline rate of monomer content and main peak content under different pH and excipients

| | SEC-HPLC | CEX-HPLC |
|---|---|---|
| Preparation number | Monomer content decline rate (%/week) | Main peak content decline rate (%/week) |
| 2-1-1 | 1.0 | 2.6 |
| 2-1-2 | 1.9 | 3.4 |
| 2-1-3 | / | / |
| 2-1-4 | 0.4 | 4.4 |
| 2-1-5 | 0.2 | 4.0 |
| 2-1-6 | 0.2 | 2.8 |
| 2-2-1 | 1.3 | 3.8 |
| 2-2-2 | 2.5 | 4.1 |
| 2-2-3 | 0.2 | 4.0 |
| 2-2-4 | 1.5 | 5.8 |
| 2-2-5 | 0.5 | 4.7 |
| 2-2-6 | 0.2 | 4.2 |
| 2-3-1 | 0.6 | 3.8 |

(continued)

| Preparation number | Monomer content decline rate (%/week) | Main peak content decline rate (%/week) |
|---|---|---|
| 2-3-2 | 0.9 | 4.1 |
| 2-3-3 | 0.7 | 4.2 |
| 2-3-4 | 0.7 | 4.3 |
| 2-3-5 | 0.4 | 4.2 |
| 2-3-6 | 0.5 | 4.1 |
| Notes: "j" denotes no determination. | | |

The results of appearance and visible particulate of the samples in the preparations containing different excipients under a high temperature condition of 40°C $\pm$ 2°C showed that a precipitate appeared in preparation 2-1-3, and other preparation samples were normal, indicating that the acetate buffer solution with pH 5.0 containing the excipient mannitol was unstable compared with the buffer solution with other pH containing other excipients.

Under three different pH conditions, the monomer purity, determined by SEC-HPLC, in the preparation containing sodium chloride and arginine hydrochloride declined rapidly, and the monomer purity, determined by SEC-HPLC, in the preparation with pH 5.5 containing sorbitol (preparation 2-2-4) also declined rapidly; at pH 5.0, sucrose and trehalose (preparations 2-1-5 and 2-1-6) were superior to other excipients, and the monomer purity decline rate was only 0.2%/week; and at pH 5.5, mannitol and trehalose (preparations 2-2-3 and 2-2-6) showed better performance, and the monomer purity decline rate was only 0.2%/week. Comparing the decline rates determined by SEC-HPLC with the decline rates determined by CEX-HPLC, it was found that preparation 2-1-6 showed the best performance, and the main peak content decline rate was only 2.8%/week. See Table 5 for details.

Based on comprehensive analysis of various data, the preparation containing the excipient mannitol (preparation 2-1-3) had protein precipitate at pH 5.0, and was normal at both pH 5.5 and pH 6.0, indicating that the excipient mannitol was relatively sensitive to pH. Preparation 2-1-6 showed the best overall performance, followed by preparation 2-1-5 and preparation 2-2-6. The excipients trehalose and sucrose showed better performance under different pH conditions. Therefore, the selection of trehalose and sucrose as excipients was more beneficial to the stability of the product.

Example 3: Experiments for screening stabilizers (excipients) and surfactants

The addition of a surfactant to a liquid preparation is often used to protect a protein, e.g., an antibody, from air/solution interface induced stress and solution/surface induced stress during storage to reduce aggregation of the antibody or to minimize the formation of particulate matter in a preparation, which is beneficial to the stability of the physicochemical properties of the antibody. To a preparation containing 20 mM acetate buffer and 40 mg/ml antibody hu20 were added trehalose, sucrose or a combination of trehalose or sucrose and sodium chloride, and polysorbate 20 or polysorbate 80 with different concentrations, respectively, to examine the effects of the above-mentioned different excipients and surfactants on stability. The information of specific preparations is as shown in Table 6. The preparations were placed at 40°C $\pm$ 2°C, and appearance and visible particulate detection, SEC-HPLC and CEX-HPLC were performed at week 0, week 2 and week 4. The results are as shown in Table 7.

Table 6: Information of preparations in experiments for screening excipients and surfactants

| Preparation number | Buffer solution | pH | Excipient 1 | Excipient 2 | Polysorbate |
|---|---|---|---|---|---|
| 3-1 | 20 mM acetate buffer (glacial acetic acid-sodium acetate) | 5.5 | 220 mM sucrose | / | 0.02% TW-80 |
| 3-2 | | | 220 mM trehalose | / | 0.02% TW-80 |
| 3-3 | | | 140 mM sucrose | 50 mM sodium chloride | 0.02% TW-80 |
| 3-4 | | | 140 mM trehalose | 50 mM sodium chloride | 0.02% TW-80 |
| 3-5 | | | 220 mM sucrose | / | 0.04%TW-80 |
| 3-6 | | | 220 mM sucrose | / | 0.08%TW-80 |
| 3-7 | | | 220 mM sucrose | / | 0.02%TW-20 |
| 3-8 | | | 220 mM sucrose | / | 0.04%TW-20 |
| 3-9 | | | 220 mM sucrose | / | 0.08%TW-20 |

Notes: "j" denotes no addition.

Table 7: Decline rate of monomer content and main peak content under different excipients and surfactants

| Preparation number | SEC-HPLC | CEX-HPLC |
|---|---|---|
| | Monomer content decline rate (%/week) | Main peak content decline rate (%/week) |
| 3-1 | 0.49 | 3.0 |
| 3-2 | 0.45 | 2.9 |
| 3-3 | Protein precipitate | Protein precipitate |
| 3-4 | 0.66 | 2.8 |
| 3-5 | 0.62 | 3.4 |
| 3-6 | 0.48 | 3.2 |
| 3-7 | 0.48 | 3.1 |
| 3-8 | 0.46 | 3.3 |
| 3-9 | 0.52 | 3.3 |

After preparation samples containing different excipients and surfactants were placed for 2 weeks under a high temperature condition of 40°C $\pm$ 2°C, the results of appearance and visible particulate showed that a precipitate appeared in preparation 3-3, and other preparation samples were normal.

The results detected by SEC-HPLC showed that under the accelerated condition of 40°C $\pm$ 2°C, the monomer purity of preparation 3-4 and preparation 3-5 declined rapidly, and aggregation was serious in preparation 3-3 to produce protein precipitate, indicating that the effects of single excipients sucrose and trehalose were superior to that of a combination of sucrose or trehalose and sodium chloride, wherein the preparation containing the single excipient trehalose had the best effect (preparation 3-2). The results of screening surfactants showed that the monomer of preparation 3-5 containing 0.04% Tween-80 declined rapidly, while the effects of other preparations (preparations 3-1/5/6/7/8/9) had little difference under different concentrations of Tween-20 and Tween-80. There was little overall difference in the main peak content determined by CEX-HPLC.

Comprehensive analysis showed that preparation 3-2 showed the best overall performance, i.e., 20 mM acetate buffer, 220 mM trehalose, pH 5.5, 0.02% Tween-80.

Example 4: Study on the long-term stability of preparations

Liquid pharmaceutical products containing therapeutic antibodies usually need to be stored at 2°C-8°C, so it is very important that the preparations maintain high stability during long-term storage. According to the above-mentioned screening results, the preparation containing 20 mM acetate buffer, 220 mM trehalose and 0.02% Tween-80 at pH 5.5 was used as the final preparation. This preparation was used for the subsequent production and examination of long-term and accelerated stability.

Four batches of finished products were selected and placed at 2°C-8°C for 0-36 months, and analysis and assay were performed on each sample. Stability was assessed by the following parameters: (a) visual appearance; (b) insoluble particles (OD405nm) determined by light obscuration; (c) pH; (d) molecular weight of antibody detected by CE-SDS (capillary electrophoresis-sodium dodecyl sulphate) method; (d) content of antibody monomer (quality standard: $\geq 95.0\%$), aggregate (quality standard: $\leq 5.0\%$), and fragment (quality standard: $\leq 3.0\%$) measured by SEC-HPLC; (e) content of main charge (quality standard: $\geq 50.0\%$), acidic charge (quality standard: $\leq 40.0\%$), and basic charge (quality standard: $\leq 30.0\%$) of antibody measured by CEX-HPLC; (f) binding activity of antibody detected by ELISA method (quality standard: 70%-130% of a reference product); and (g) protein content (quality standard: 36.0-44.0 mg/ml).

The results showed that the appearance, pH, insoluble particles, protein content, purity (SEC-HPLC (size exclusion chromatography-high performance liquid chromatography), CEX-HPLC (weak cation exchange high performance liquid chromatography), R-CE-SDS (reduced capillary electrophoresis-sodium dodecyl sulphate), NR-CE-SDS (non-reduced capillary electrophoresis-sodium dodecyl sulphate)) and biological activity of the 4 batches of finished products had no significant change. See Table 8 for details. The results showed that the above-mentioned 4 batches of stock solutions had very good stability during storage for 0-12 months at 2°C-8°C.

Table 8: Long-term stability data of preparations

| Detection item | Time (month) | Detection results | | | |
|---|---|---|---|---|---|
| | | Batch 1 | Batch 2 | Batch 3 | Batch 4 |
| Appearance | 0 | Nearly colourless liquid, with slight opalescence | Nearly colourless liquid, with slight opalescence | Character: liquid Colour: < yellow No. 2 standard colourimetric solution Clarity: < No. 3 turbidity standard solution | Character: liquid Colour: < yellow No. 2 standard colourimetric solution Clarity: < No. 3 turbidity standard solution |
| | 3 | Character: liquid Colour: = yellow No. 0 standard colourimetric solution Clarity: <No. 2 turbidity standard solution | Character: liquid Colour: = yellow No. 0 standard colourimetric solution Clarity: <No. 2 turbidity standard solution | ChP: the colour of the liquid is equal to YO standard colourimetric solution Clarity: ≤ No. 0.5 turbidity standard solution | ChP: the colour of the liquid is equal to YO standard colourimetric solution Clarity: ≤ No. 0.5 turbidity standard solution |
| | 6 | Character: liquid Colour: = yellow No. 0 standard colourimetric solution Clarity: <No. 1 turbidity standard solution | Character: liquid Colour: = yellow No. 0 standard colourimetric solution Clarity: <No. 1 turbidity standard solution | Character: liquid Colour: = YO standard colourimetric solution Clarity: ≤ No. 1 turbidity standard solution | Character: liquid Colour: = YO standard colourimetric solution Clarity: ≤ No. 1 turbidity standard solution |
| | 9 | Character: liquid Colour: = yellow No. 0 standard colourimetric solution Clarity: <No. 5 turbidity standard solution | Character: liquid Colour: = yellow No. 0 standard colourimetric solution Clarity: ≤ No. 6 turbidity standard solution | Character: liquid Colour: < yellow No. 2 standard colourimetric solution Clarity: <No. 3 turbidity standard solution | Character: liquid Colour: < yellow No. 2 standard colourimetric solution Clarity: <No. 3 turbidity standard solution |

(continued)

| Detection item | Time (month) | Detection results | | | |
|---|---|---|---|---|---|
| | | Batch 1 | Batch 2 | Batch 3 | Batch 4 |
| | 12 | Character: liquid Colour: equal to YO standard colourimetric solution Clarity: ≤ No. 0.5 turbidity standard solution | Character: liquid Colour: equal to YO standard colourimetric solution Clarity: <No. 0.5 turbidity standard solution | Character: liquid Colour: = YO standard colourimetric solution Clarity: <No. 3 turbidity standard solution | Character: liquid Colour: = YO standard colourimetric solution Clarity: <No. 3 turbidity standard solution |
| | 18 | Character: liquid Colour: < Y0.5 standard colourimetric solution Clarity: <No. 2 turbidity standard solution | Character: liquid Colour: < Y0.5 standard colourimetric solution Clarity: <No. 2 turbidity standard solution | NR | NR |
| | 24 | NR | NR | NR | NR |
| | 36 | NR | NR | NR | NR |
| pH | 0 | 5.5 | 5.6 | 5.6 | 5.6 |
| | 3 | 5.6 | 5.6 | 5.6 | 5.5 |
| | 6 | 5.7 | 5.7 | 5.6 | 5.6 |
| | 9 | 5.6 | 5.7 | 5.5 | 5.6 |
| | 12 | 5.6 | 5.6 | 5.6 | 5.6 |
| | 18 | 5.6 | 5.6 | NR | NR |
| | 24 | NR | NR | NR | NR |
| | 36 | NR | NR | NR | NR |
| Monomer content (%) determined by SEC-HPLC | 0 | 99.8% | 99.7% | 99.5% | 99.5% |
| | 3 | 99.4% | 99.3% | 99.5% | 99.5% |
| | 6 | 99.2% | 99.1% | 99.3% | 99.3% |
| | 9 | 99.3% | 99.1% | 99.2% | 99.3% |
| | 12 | 99.2% | 99.0% | 99.1% | 99.1% |
| | 18 | 99.1% | 99.1% | NR | NR |
| | 24 | NR | NR | NR | NR |
| | 36 | NR | NR | NR | NR |

(continued)

| Detection item | Time (month) | Detection results | | | |
|---|---|---|---|---|---|
| | | Batch 1 | Batch 2 | Batch 3 | Batch 4 |
| Aggregate content (%) determined by SEC-HPLC | 0 | 0.2% | 0.3% | 0.4% | 0.4% |
| | 3 | 0.6% | 0.7% | 0.5% | 0.5% |
| | 6 | 0.7% | 0.8% | 0.6% | 0.6% |
| | 9 | 0.7% | 0.9% | 0.7% | 0.7% |
| | 12 | 0.7% | 0.9% | 0.8% | 0.8% |
| | 18 | 0.8% | 0.8% | NR | NR |
| | 24 | NR | NR | NR | NR |
| | 36 | NR | NR | NR | NR |
| Fragment content (%) determined by SEC-HPLC | 0 | Not detected | Not detected | < 0.1% | < 0.1% |
| | 3 | Not detected | Not detected | Not detected | Not detected |
| | 6 | < 0.1% | < 0.1% | < 0.1% | < 0.1% |
| | 9 | Not detected | Not detected | 0.1% | 0.1% |
| | 12 | 0.1% | 0.1% | 0.2% | 0.2% |
| | 18 | 0.1% | 0.1% | NR | NR |
| | 24 | NR | NR | NR | NR |
| | 36 | NR | NR | NR | NR |
| Main peak content (%) determined by CEX-HPLC | 0 | 76.6% | 77.3% | 78.8% | 77.2% |
| | 3 | 75.9% | 75.0% | 82.4% | 78.7% |
| | 6 | 77.3% | 76.7% | 79.5% | 77.9% |
| | 9 | 75.9% | 75.4% | 78.0% | 75.9% |
| | 12 | 81.1% | 80.7% | 79.6% | 77.3% |
| | 18 | 78.4% | 78.1% | NR | NR |
| | 24 | NR | NR | NR | NR |
| | 36 | NR | NR | NR | NR |
| Acidic peak content (%) determined by CEX-HPLC | 0 | 20.6% | 19.1% | 18.0% | 19.8% |
| | 3 | 19.3% | 19.5% | 14.6% | 18.4% |
| | 6 | 18.7% | 19.0% | 17.3% | 18.9% |
| | 9 | 18.8% | 19.0% | 16.6% | 18.4% |
| | 12 | 15.7% | 15.7% | 16.8% | 19.1% |
| | 18 | 16.4% | 16.4% | NR | NR |
| | 24 | NR | NR | NR | NR |
| | 36 | NR | NR | NR | NR |

(continued)

| Detection item | Time (month) | Detection results | | | |
|---|---|---|---|---|---|
| | | Batch 1 | Batch 2 | Batch 3 | Batch 4 |
| Basic peak content (%) determined by CEX-HPLC | 0 | 2.8% | 3.6% | 3.2% | 3.0% |
| | 3 | 4.8% | 5.1% | 2.9% | 2.9% |
| | 6 | 4.0% | 4.3% | 3.2% | 3.1% |
| | 9 | 5.3% | 5.6% | 5.5% | 5.6% |
| | 12 | 3.2% | 3.6% | 3.5% | 3.5% |
| | 18 | 5.2% | 5.4% | NR | NR |
| | 24 | NR | NR | NR | NR |
| | 36 | NR | NR | NR | NR |
| Content (%) determined by R-CE-SDS | 0 | 99.8% | 99.8% | 99.7% | 99.7% |
| | 3 | 99.7% | 99.6% | 99.6% | 99.5% |
| | 6 | 99.7% | 99.4% | 99.7% | 99.6% |
| | 9 | 99.6% | 99.4% | 99.5% | 99.6% |
| | 12 | 99.4% | 99.4% | 99.5% | 99.6% |
| | 18 | 99.6% | 99.2% | NR | NR |
| | 24 | NR | NR | NR | NR |
| | 36 | NR | NR | NR | NR |
| Content (%) determined by NR-CE-SDS | 0 | 98.3% | 98.0% | 97.6% | 97.8% |
| | 3 | 96.8% | 95.9% | 97.5% | 97.3% |
| | 6 | 98.2% | 97.5% | 96.4% | 97.4% |
| | 9 | 97.3% | 97.1% | 96.0% | 96.0% |
| | 12 | 97.4% | 97.0% | 97.2% | 97.0% |
| | 18 | 98.1% | 97.5% | NR | NR |
| | 24 | NR | NR | NR | NR |
| | 36 | NR | NR | NR | NR |
| Biological activity (Binding ELISA method), % | 0 | 109% | 94% | 101% | 106% |
| | 3 | 104% | 107% | 103% | 101% |
| | 6 | 104% | 106% | 98% | 101% |
| | 9 | 106% | 110% | 107% | 103% |
| | 12 | 99% | 106% | 109% | 106% |
| | 18 | 100% | 97% | NR | NR |
| | 24 | NR | NR | NR | NR |
| | 36 | NR | NR | NR | NR |

(continued)

| Detection item | Time (month) | Detection results | | | |
|---|---|---|---|---|---|
| | | Batch 1 | Batch 2 | Batch 3 | Batch 4 |
| Protein content (Ultraviolet spectrophotometry) | 0 | 40.8 mg/ml | 40.5 mg/ml | 39.2 mg/ml | 38.6 mg/ml |
| | 3 | 40.2 mg/ml | 40.0 mg/ml | 39.7 mg/ml | 39.2 mg/ml |
| | 6 | 39.6 mg/ml | 39.6 mg/ml | 40.3 mg/ml | 40.0 mg/ml |
| | 9 | 40.6 mg/ml | 40.8 mg/ml | 39.5 mg/ml | 38.7 mg/ml |
| | 12 | 40.2 mg/ml | 39.3 mg/ml | 39.7 mg/ml | 39.3 mg/ml |
| | 18 | 40.5 mg/ml | 40.2 mg/ml | NR | NR |
| | 24 | NR | NR | NR | NR |
| | 36 | NR | NR | NR | NR |
| Insoluble particles | 0 | $\geq 10\ \mu m$: 42 particles/tube, $\geq 25\ \mu m$: 4 particles/tube | $\geq 10\ \mu m$: 5 particles/tube, $\geq 25\ \mu m$: 2 particles/tube | $\geq 10\ \mu m$: 14 particles/tube, $\geq 25\ \mu m$: 4 particles/tube | $\geq 10\ \mu m$: 12 particles/tube, $\geq 25\ \mu m$: 0 particle/tube |
| | 3 | $\geq 10\ \mu m$: 2 particles/tube $\geq 25\ \mu m$: 0 particle/tube | $\geq 10\ \mu m$: 4 particles/tube $\geq 25\ \mu m$: 1 particle/tube | $\geq 10\ \mu m$: 12 particles/tube, $\geq 25\ \mu m$: 2 particles/tube | ChP: $\geq 10\ \mu m$: 3 particles/tube, $\geq 25\ \mu m$: 0 particle/tube |
| | 6 | $\geq 10\ \mu m$: 15 particles/tube $\geq 25\ \mu m$: 2 particles/tube | $\geq 10\ \mu m$: 15 particles/tube $\geq 25\ \mu m$: 3 particles/tube | $\geq 10\ \mu m$: 11 particles/tube $\geq 25\ \mu m$: 6 particles/tube | $\geq 10\ \mu m$: 2 particles/tube $\geq 25\ \mu m$: 2 particles/tube |
| | 9 | $\geq 10\ \mu m$: 15 particles/tube $\geq 25\ \mu m$: 10 particles/tube | $\geq 10\ \mu m$: 15 particles/tube $\geq 25\ \mu m$: 11 particles/tube | $\geq 10\ \mu m$: 33 particles/tube, $\geq 25\ \mu m$: 7 particles/tube | $\geq 10\ \mu m$: 23 particles/tube, $\geq 25\ \mu m$: 4 particles/tube |
| | 12 | $\geq 10\ \mu m$: 1 particle/tube, $\geq 25\ \mu m$: 0 particle/tube | $\geq 10\ \mu m$: 0 particle/tube, $\geq 25\ \mu m$: 0 particle/tube | $\geq 10\ \mu m$: 16 particles/tube, $\geq 25\ \mu m$: 3 particles/tube | $\geq 10\ \mu m$: 7 particles/tube, $\geq 25\ \mu m$: 3 particles/tube |
| | 18 | $\geq 10\ \mu m$: 4 particles/tube, $\geq 25\ \mu m$: 2 particles/tube | $\geq 10\ \mu m$: 4 particles/tube, $\geq 25\ \mu m$: 1 particle/tube | NR | NR |
| | 24 | NR | NR | NR | NR |
| | 36 | NR | NR | NR | NR |
| Notes: NR denotes that the time point has not been reached. | | | | | |

Example 5: Study of accelerated stability of preparations

Finished products were used for studying accelerated stability. After samples were placed at 25°C $\pm$ 2°C and 60% $\pm$ 5% relative humidity (RH) for 0-6 months, each sample was subjected to analysis and assay.
As shown in Table 9, this finished preparation had high stability against protein degradation, and the degradation kinetic parameters measured at 25°C $\pm$ 2°C met the requirements of storage in a room temperature environment for up to 6 months.

Table 9: Accelerated stability data of preparations

| Detection item | Time (month) | Detection results | | | |
|---|---|---|---|---|---|
| | | Batch 1 | Batch 2 | Batch 3 | Batch 4 |
| Appearance | 0 | Nearly colourless liquid, with slight opalescence | Nearly colourless liquid, with slight opalescence | Character: liquid Colour: < yellow No. 2 standard colourimetric solution Clarity: < No. 3 turbidity standard solution | Character: liquid Colour: < yellow No. 2 standard colourimetric solution Clarity: < No. 3 turbidity standard solution |
| | 1 | Character: liquid Colour: = yellow No. 0 standard colourimetric solution Clarity: <No. 2 turbidity standard solution | Character: liquid Colour: = yellow No. 0 standard colourimetric solution Clarity: <No. 2 turbidity standard solution | Character: liquid Colour: < yellow No. 2 standard colourimetric solution Clarity: < No. 3 turbidity standard solution | Character: liquid Colour: < yellow No. 2 standard colourimetric solution Clarity: < No. 3 turbidity standard solution |
| | 2 particles/ tube | Character: liquid Colour: = yellow No. 0 standard colourimetric solution Clarity: <No. 2 turbidity standard solution | Character: liquid Colour: = yellow No. 0 standard colourimetric solution Clarity: <No. 2 turbidity standard solution | Character: liquid Colour: < yellow No. 2 standard colourimetric solution Clarity: < No. 3 turbidity standard solution | Character: liquid Colour: < yellow No. 2 standard colourimetric solution Clarity: < No. 3 turbidity standard solution |
| | 3 | Character: liquid Colour: = yellow No. 0 standard colourimetric solution Clarity: <No. 2 turbidity standard solution | Character: liquid Colour: = yellow No. 0 standard colourimetric solution Clarity: <No. 2 turbidity standard solution | ChP: the colour of the liquid is equal to YO standard colourimetric solution Clarity: $\leq$ No. 0.5 turbidity standard solution | ChP: the colour of the liquid is equal to YO standard colourimetric solution Clarity: $\leq$ No. 0.5 turbidity standard solution |

(continued)

| Detection item | Time (month) | Detection results | | | |
|---|---|---|---|---|---|
| | | Batch 1 | Batch 2 | Batch 3 | Batch 4 |
| | 6 | Character: liquid | Character: liquid | Character: liquid | Character: liquid |
| | | Colour: = yellow No. 0 standard colourimetric solution Clarity: <No. 1 turbidity standard solution | Colour: = yellow No. 0 standard colourimetric solution Clarity: <No. 1 turbidity standard solution | Colour: = YO standard colourimetric solution Clarity: ≤ No. 1 turbidity standard solution | Colour: = YO standard colourimetric solution Clarity: ≤ No. 1 turbidity standard solution |
| pH | 0 | 5.5 | 5.6 | 5.6 | 5.6 |
| | 1 | 5.6 | 5.6 | 5.6 | 5.6 |
| | 2 | 5.6 | 5.6 | 5.6 | 5.6 |
| | 3 | 5.6 | 5.6 | 5.6 | 5.6 |
| | 6 | 5.8 | 5.7 | 5.6 | 5.6 |
| Monomer content (%) determined by SEC-HPLC | 0 | 99.8% | 99.7% | 99.5% | 99.5% |
| | 1 | 99.4% | 99.1% | 99.3% | 99.3% |
| | 2 | 99.2% | 99.0% | 99.1% | 99.1% |
| | 3 | 99.1% | 98.9% | 98.9% | 98.8% |
| | 6 | 98.7% | 98.5% | 98.8% | 98.8% |
| Aggregate content (%) determined by SEC-HPLC | 0 | 0.2% | 0.3% | 0.4% | 0.4% |
| | 1 | 0.6% | 0.8% | 0.7% | 0.7% |
| | 2 | 0.8% | 1.0% | 0.8% | 0.8% |
| | 3 | 0.9% | 1.1% | 0.9% | 0.9% |
| | 6 | 1.2% | 1.4% | 1.1% | 1.1% |
| Fragment content (%) determined by SEC-HPLC | 0 | Not detected | Not detected | < 0.1% | < 0.1% |
| | 1 | Not detected | < 0.1% | < 0.1% | < 0.1% |
| | 2 | < 0.1% | < 0.1% | 0.1% | 0.1% |
| | 3 | < 0.1% | < 0.1% | 0.3% | 0.3% |
| | 6 | 0.1% | 0.1% | < 0.1% | < 0.1% |
| Main peak content (%) determined by CEX-HPLC | 0 | 76.6% | 77.3% | 78.8% | 77.2% |
| | 1 | 75.8% | 75.0% | 77.1% | 75.5% |
| | 2 | 74.8% | 74.1% | 79.2% | 78.0% |
| | 3 | 69.2% | 69.4% | 80.1% | 78.1% |
| | 6 | 67.6% | 67.0% | 77.1% | 75.4% |

(continued)

| Detection item | Time (month) | Detection results | | | |
|---|---|---|---|---|---|
| | | Batch 1 | Batch 2 | Batch 3 | Batch 4 |
| Acidic peak content (%) determined by CEX-HPLC | 0 | 20.6% | 19.1% | 18.0% | 19.8% |
| | 1 | 19.6% | 20.0% | 17.6% | 19.4% |
| | 2 | 20.0% | 20.4% | 15.4% | 16.7% |
| | 3 | 24.8% | 24.4% | 16.2% | 17.7% |
| | 6 | 26.8% | 27.3% | 18.0% | 19.3% |
| Basic peak content (%) determined by CEX-HPLC | 0 | 2.8% | 3.6% | 3.2% | 3.0% |
| | 1 | 4.0% | 5.0% | 5.3% | 5.1% |
| | 2 | 5.2% | 5.5% | 5.4% | 5.4% |
| | 3 | 6.0% | 6.1% | 3.7% | 4.2% |
| | 6 | 5.6% | 5.7% | 4.9% | 5.3% |
| Content (%) determined by R-CE-SDS | 0 | 99.8% | 99.8% | 99.7% | 99.7% |
| | 1 | 99.5% | 99.5% | 99.6% | 99.5% |
| | 2 | 99.3% | 99.2% | 99.4% | 99.4% |
| | 3 | 99.4% | 99.6% | 99.4% | 99.3% |
| | 6 | 99.2% | 99.1% | 98.7% | 99.2% |
| Content (%) determined by NR-CE-SDS | 0 | 98.3% | 98.0% | 97.6% | 97.8% |
| | 1 | 97.3% | 97.6% | 97.3% | 97.4% |
| | 2 | 97.5% | 96.3% | 96.6% | 96.7% |
| | 3 | 95.8% | 95.2% | 96.7% | 96.5% |
| | 6 | 96.4% | 96.6% | 95.5% | 95.3% |
| Biological activity (Binding ELISA method), % | 0 | 109% | 94% | 101% | 106% |
| | 1 | 93% | 94% | 106% | 106% |
| | 2 | 102% | 96% | 105% | 103% |
| | 3 | 111% | 123% | 94% | 98% |
| | 6 | 107% | 94% | 98% | 96% |
| Protein content (Ultraviolet spectrophotometry) | 0 | 40.8 mg/ml | 40.5 mg/ml | 39.2 mg/ml | 38.6 mg/ml |
| | 1 | 41.5 mg/ml | 39.7 mg/ml | 39.3 mg/ml | 38.8 mg/ml |
| | 2 | 40.4 mg/ml | 39.7 mg/ml | 39.8 mg/ml | 39.3 mg/ml |
| | 3 | 40.6 mg/ml | 40.0 mg/ml | 39.0 mg/ml | 39.1 mg/ml |
| | 6 | 38.9 mg/ml | 39.5 mg/ml | 40.3 mg/ml | 40.1 mg/ml |

(continued)

| Detection item | Time (month) | Detection results | | | |
|---|---|---|---|---|---|
| | | Batch 1 | Batch 2 | Batch 3 | Batch 4 |
| Insoluble particles | 0 | ≥ 10 μm: 42 particles/tube, ≥ 25 μm: 4 particles/tube | ≥ 10 μm: 5 particles/tube, ≥ 25 μm: 2 particles/tube | ≥ 10 μm: 14 particles/tube, ≥ 25 μm: 4 particles/tube | ≥ 10 μm: 12 particles/tube, ≥ 25 μm: 0 particle/tube |
| | 1 | ≥ 10 μm: 18 particles/tube ≥ 25 μm: 2 particles/tube | ≥ 10 μm: 2 particles/tube ≥ 25 μm: 0 particle/tube | ≥ 10 μm: 6 particles/tube, ≥ 25 μm: 2 particles/tube | ≥ 10 μm: 9 particles/tube, ≥ 25 μm: 3 particles/tube |
| | 2 | ≥ 10 μm: 10 particles/tube ≥ 25 μm: 1 particle/tube | ≥ 10 μm: 2 particles/tube ≥ 25 μm: 0 particle/tube | >10 μm: 10 particles/tube, ≥ 25 μm: 1 particle/tube | ≥ 10 μm: 6 particles/tube, ≥ 25 μm: 0 particle/tube |
| | 3 | ≥ 10 μm: 6 particles/tube ≥ 25 μm: 0 particle/tube | ≥ 10 μm: 7 particles/tube ≥ 25 μm: 0 particle/tube | ChP: ≥ 10 μm: 12 particles/tube, ≥ 25 μm: 1 particle/tube | ChP: ≥ 10 μm: 12 particles/tube, ≥ 25 μm: 3 particles/tube |
| | 6 | ≥ 10 μm: 15 particles/tube ≥ 25 μm: 4 particles/tube | ≥ 10 μm: 15 particles/tube ≥ 25 μm: 5 particles/tube | ≥ 10 μm: 4 particles/tube ≥ 25 μm: 0 particle/tube | ≥ 10 μm: 12 particles/tube ≥ 25 μm: 1 particle/tube |

Example 6: Confirmation of influencing factors and formula of preparation

An antibody stock solution was used; preparations as shown in Table 10 were prepared; and the stability of samples under repeated freeze-thaw conditions was examined to confirm formulation conditions, with a concentration of antibody hu20 being 40 mg/ml. The examination indexes are as shown in Table 11.

Table 10: Information of influencing factors of preparations

| Preparation number | Buffer solution | pH | Excipient 1 | Excipient 2 | Polysorbate |
|---|---|---|---|---|---|
| 3-1 | 20 mM acetate buffer (glacial acetic acid-sodium acetate) | 5.5 | 220 mM sucrose | / | 0.02% TW-80 |
| 3-2 | | | 220 mM trehalose | / | 0.02% TW-80 |
| 3-3 | | | 140 mM sucrose | 50 mM sodium chloride | 0.02% TW-80 |
| 3-4 | | | 140 mM trehalose | 50 mM sodium chloride | 0.02% TW-80 |
| Notes: "j" denotes no addition. | | | | | |

Table 11: Examination condition, time point and detection item

| Examination condition | Time point | | | Detection item |
|---|---|---|---|---|
| | 0 W | 2 W | 4 W | |
| Repeated freeze-thaw | √ | -40°C to room temperature, three consecutive times | | Appearance, SEC-HPLC, Binding ELISA |

The results showed that after being placed at ≤ -40°C for at least 5 hours and being completely frozen, the preparations were taken out and completely thawed at room temperature. After repeated freeze-thaw three times, there were no significant changes in appearance and the results of SEC-HPLC and Binding ELISA (relative binding activity), showing good stability of the preparations. See Table 12 for details.

Table 12: Screening results of repeated freeze-thaw

| Preparation number | Appearance | SEC-HPLC | | | Relative binding activity, % |
|---|---|---|---|---|---|
| | | Aggregate, % | Monomer, % | Fragment, % | |
| 3-1 | Normal | 0.3 | 99.7 | 0.0 | 106.4 |
| 3-2 | Normal | 0.3 | 99.7 | 0.0 | 110.2 |
| 3-3 | Normal | 0.3 | 99.7 | 0.0 | 114.3 |
| 3-4 | Normal | 0.3 | 99.7 | 0.0 | 109.3 |

In summary, the stability of recombinant humanized anti-TIGIT monoclonal antibody hu20 is explored and studied and the optimal formula of the liquid preparation is determined by examining different buffer solutions, different pH conditions, different antibody concentrations, and different compositions of excipients and surfactants. According to the above-mentioned experiments, an acetate buffer is selected for adjusting the pH of the antibody of the present invention; trehalose is selected for adjusting the osmotic pressure of the preparation; and polysorbate 80 is added to increase the solubility of the preparation.

Example 7: Effect of humanized anti-TIGIT antibody on T cell activity

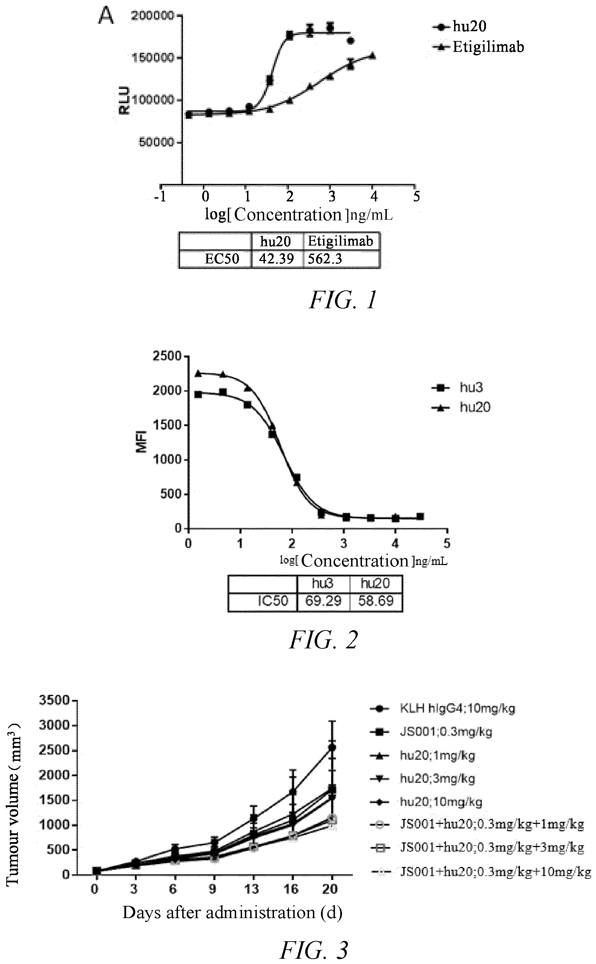

CHO cells stably expressing hPVRL2 were plated into a 96-well plate at a density of $5 \times 10^4$ cells per well, and cultured overnight at 37°C and 7% $CO_2$. The cell supernatant was removed. 40 $\mu$L of a diluent of anti-TIGIT antibody (hu20 or MBSA43) (starting concentration being 10 ug/ml, 3-fold titration) was added to each well, and 40 $\mu$L of Jurkat reporter cells capable of persistently expressing hTIGIT/NFAT-luciferase were added, with a total cell number of $1 \times 10^5$. The cells were cultured for 6 h at 37°C and 7% $CO_2$, to which a luciferase reagent was added. Luminescence value was detected by a microplate reader.
Among them, MBSA43 is a commercial anti-human TIGIT antibody (Etigilimab), purchased from ThermoFisher, Catalog No.: 16-9500-82.
The anti-TIGIT antibody of the present invention was prepared according to the formula of preparation 3-2.
Data were analysed using GraphPad Prism 5 to calculate the $EC_{50}$ value of T cells activated by the anti-TIGIT antibody, thereby assessing the effect of the anti-TIGIT antibody on T cell activity.
As shown in FIG. 1, antibody hu20 may significantly enhance the fluorescence signal, i.e., promoting the activation of T blood cells, with an $EC_{50}$ of 42.39 ng/mL; and the activation of T blood cells by antibody hu20 is significantly superior to that by control antibody Etigilimab.

Example 8: Detection of the blocking effect of antibody on the binding of hTIGIT to its ligand hPVR by FACS

The blocking effect of an antibody on the binding of hTIGIT to its ligand hPVR, etc. was detected using competitive fluorescence-activated cell sorting (FACS) assay. Briefly, diluents of antibodies with different concentrations (starting concentration being 30 $\mu$g/ml, 3-fold titration) were mixed with PVR-mFC (1 ug/ml, 50 $\mu$L), and the mixture was incubated at room temperature for 30 minutes. The mixture was then incubated with a cell suspension (293F-hPVR stable transformation cell line, $2.5 \times 10^4$ cells/well) at 37°C for 15 minutes, and eluted with PBS 3 times. Then 100 $\mu$L of goat anti-human IgG-PE antibody was added, and the resulting mixture was incubated in the dark for 30 min. Detection was performed by FACS after elution with PBS 3 times. Living cells were gated from FSC/SSC and their geometric mean fluorescence was measured.

The anti-TIGIT antibody of the present invention was prepared according to the formula of preparation 3-2.

As shown in FIG. 2, the humanized antibodies of the present invention may effectively block the binding of TIGIT to PVR on the surface of cells.

Example 9: Detection of the binding of humanized antibodies to different species of TIGIT by BIACORE

The affinity and binding kinetics between the antibodies of the present invention and TIGIT were detected using Biacore T200 (GE). First, Series S chip CM5 (GE) was loaded onto an instrument, and 40 $\mu$g/mL goat anti-human Fc fragment antibody (Jackson ImmuneResearch) dissolved in a sodium acetate-acetate buffer (pH 5.0) was prepared to be coupled to the surface of the chip. The buffer used for detecting the binding of an antibody to an antigen was HBS-EP+ from GE Healthcare Life Sciences. Antibodies hu3 and hu20 were diluted to 8 $\mu$g/mL, respectively, and captured on the surface of the chip. Antigen hTIGIT Fc was diluted to 20 nM, and injected into the surface of the chip at a flow rate of 30 $\mu$L/min for 180 s, and the binding signal and dissociation signal of the antibody and the antigen were detected. The resulting data were analysed using Biacore T200 Evaluation Software 3.0 software with a 1 : 1 binding model. The kinetic constants, namely binding rate ka (1/Ms), dissociation rate kd (1/s) and equilibrium dissociation constant $K_D$ (M), of the binding between the antibody and the antigen were obtained by fitting, and were as shown in Table 13 below.

The anti-TIGIT antibody of the present invention was prepared according to the formula of preparation 3-2.

Table 13: Affinity of antibody to TIGIT-Fc

| Antibody | Antigen | ka (1/Ms) | kd (1/s) | $K_D$ (M) |
|----------|---------|-----------|----------|-----------|
| hu3 | Cyno TIGIT-Fc | 2.11E+05 | 3.00E-04 | 1.42E-09 |
| hu20 | Cyno TIGIT-Fc | 2.26E+05 | 3.29E-04 | 1.45E-09 |
| hu3 | hTIGIT-Fc | 9.35E+05 | 1.21E-04 | 1.30E-10 |
| hu20 | hTIGIT-Fc | 7.77E+05 | 1.63E-04 | 2.09E-10 |

The results showed that the antibodies of the present invention had high affinity to human and cynomolgus TIGIT, with a $K_D$ value of the antibodies to human TIGIT as low as 0.13 nM.

Example 10: Inhibitory effect of humanized antibody on tumour growth in mice

This study involves the establishment of an MC38 colon cancer animal model in B-hPD-1/hTIGIT humanized mice (purchased from Biocytogen Jiangsu Co., Ltd.; female) and the study of the synergistic anti-tumour effect of an anti-TIGIT antibody and its combined administration with an anti-PD1 antibody.

MC38 cells resuspended with PBS were inoculated subcutaneously in the right dorsal side of B-hPD-1/hTIGIT humanized mice at a concentration of $5 \times 10^5$ cells/0.1 mL, 0.1 mL per mouse. When the mean tumour volume reached 80-100 mm$^3$, suitable mice were selected according to the tumour volume and body weight of the mice and evenly distributed into 8 experimental groups, with 8 mice in each group. Administration was started on the day of grouping. See Table 14 below for specific administration regimen.

Table 14: Administration regimen

| Group | Antibody administered | Dose, (mg/kg)" | Administration route | Administration frequency[b] | Number of administrations |
|-------|----------------------|----------------|----------------------|------------------------------|----------------------------|
| 1 | KLH hIgG4 | 10 | i.p | BIW | 6 |
| 2 | JS001 | 0.3 | i.p | BIW | 6 |
| 3 | hu20 | 1 | i.p | BIW | 6 |
| 4 | hu20 | 3 | i.p | BIW | 6 |
| 5 | hu20 | 10 | i.p | BIW | 6 |
| 6 | JS001+hu20 | 0.3+1 | i.p | BIW | 6 |
| 7 | JS001+hu20 | 0.3+3 | i.p | BIW | 6 |

(continued)

| Group | Antibody administered | Dose, (mg/kg)" | Administration route | Administration frequency[b] | Number of administrations |
|---|---|---|---|---|---|
| 8 | JS001+hu20 | 0.3+10 | i.p | BIW | 6 |

Notes: a: Administration volume is calculated according to 10 $\mu$L per g of the body weight of experimental animals;
b: BIW refers to bis in week;
i.p: intraperitoneal injection;
KLH hIgG4: Negative control antibody
JS001: Anti-PD-1 antibody developed independently by SHANGHAI JUNSHI BIOSCIENCES CO., LTD. (CN 2013102582892, antibody 38).

The anti-TIGIT antibody of the present invention was prepared according to the formula of preparation 3-2.

Throughout the study period, tumour volumes and animal body weights were measured twice a week from day 6 (before administration), with continuous monitoring for 3 weeks. The long diameter (L) and short diameter (W) of the tumour were measured using a vernier calliper, and the tumour volume (V) was calculated according to the following formula: $V = L \times W^2/2$. The tumour volumes of mice from each group were plotted versus time. Analysis of variance (ANOVA) was used to determine statistical significance. $P < 0.05$ was considered statistically significant in all analyses.

At the end of the experiment, i.e., day 26, the mice were euthanized. The tumour tissues were isolated, photographed and weighed, the tumour weight and volume (terminal tumour volume) of each group of mice were measured, and the relative tumour growth inhibition rate (TGI (%)) was calculated.

Result:

(1) As shown in FIG. 3, in terms of single medication, at 3 weeks after administration, compared with administration group 1 (KLH hIgG4; 10 mg/kg), administration group 2 (JS001; 0.3 mg/kg), administration group 3 (hu20; 1 mg/kg), administration group 4 (hu20; 3 mg/kg) and administration group 5 (hu20; 10 mg/kg) showed significant inhibitory effect on tumour growth in MC38 tumour-bearing mice, with reduced tumour volume and slowed tumour growth. In terms of combined medication, compared with the single administration group, the combined administration group (group 6 vs group 3/group 2; group 7 vs group 4/group 2; group 8 vs group 5/group 2) showed more significant inhibitory effect on tumour growth in MC38 tumour-bearing mice, that is, the combined administration of the anti-TIGIT monoclonal antibody hu20 of the present invention and anti-PD-1 monoclonal antibody JS001 showed good synergistic anti-tumour effect.

(2) In the present invention, the relative tumour growth inhibition rate in each group of mice at the end of the experiment, i.e., day 26 (after tumour implantation), was also calculated. The formula for calculating the relative tumour growth inhibition rate is as follows:

$$\text{Relative tumour growth inhibition rate TGI (\%)} = [1-(T_i-T_0)/(V_i-V_0)] \times 100\%$$

wherein, $T_i-T_0$ = terminal tumour volume after administration in administration group-tumour volume before administration in administration group; $V_i-V_0$ = terminal tumour volume after administration in control group-tumour volume before administration in control group (tumour volume before administration, i.e., day 6)

The calculation results are as shown in Table 15 below.

Table 15: Effect of anti-TIGIT antibody on tumour tissue growth in tumour-bearing mice (mm$^3$, Mean $\pm$ SD, n = 8)

| Group | Antibody administered; Dose | Mean tumour volume at the end of the experiment (mm$^3$, Mean $\pm$ SD, n = 8) | TGI (%) |
|---|---|---|---|
| 1 | KLH hIgG4; 10 mg/kg | 2562 $\pm$ 529 | N/A |
| 2 | JS001; 0.3 mg/kg | 1713 $\pm$ 876 | 34.3% |
| 3 | hu20; 1 mg/kg | 1739 $\pm$ 956 | 33.2% |

(continued)

| Group | Antibody administered; Dose | Mean tumour volume at the end of the experiment (mm$^3$, Mean $\pm$ SD, n = 8) | TGI (%) |
|---|---|---|---|
| 4 | hu20; 3 mg/kg | 1540 $\pm$ 809 | 41.3% |
| 5 | hu20; 10 mg/kg | 1555 $\pm$ 549 | 40.7% |
| 6 | JS001+hu20; 0.3 mg/kg + 1 mg/kg | 1153 $\pm$ 617 | 56.9% |
| 7 | JS001+hu20; 0.3 mg/kg + 3 mg/kg | 1101 $\pm$ 712 | 59.0% |
| 8 | JS001+hu20; 0.3 mg/kg + 10 mg/kg | 990 $\pm$ 609 | 63.5% |
| Mean $\pm$ SD: Mean $\pm$ standard deviation. | | | |

The results showed that at the end of the experiment (day 26 after tumour implantation), the relative tumour growth inhibition rate TGI (%) of the combined administration group of anti-TIGIT antibody hu20 and anti-PD-1 antibody JS001 was significantly higher than that of the single administration group hu20 or JS001 (group 6 vs group 3/group 2; group 7 vs group 4/group 2; group 8 vs group 5/group 2).

**Claims**

1.  A pharmaceutical composition, comprising:

    (1) a buffer solution; and
    (2) an anti-TIGIT antibody or an antigen-binding fragment thereof;

    wherein the anti-TIGIT antibody or the antigen-binding fragment thereof comprises HCDR1, HCDR2, and HCDR3 having amino acid sequences as respectively represented by SEQ ID NO: 1, SEQ ID NO:2, and SEQ ID NO:3, and LCDR1, LCDR2, and LCDR3 having amino acid sequences as respectively represented by SEQ ID NO:4, SEQ ID NO:5, and SEQ ID NO:6; wherein the pH of the pharmaceutical composition is about 5.0-6.5, preferably about 5.0-6.0.

2.  The pharmaceutical composition of claim 1, wherein the buffer solution is selected from an acetate buffer, a citrate buffer and a histidine buffer; preferably, the buffer solution is an acetate buffer.

3.  The pharmaceutical composition of claim 2, wherein the concentration of the buffer solution is about 10-50 mM; preferably, the concentration of the buffer solution is about 10-30 mM.

4.  The pharmaceutical composition of claim 2, wherein the pH of the buffer solution is about 5.0-6.5, preferably about 5.0-6.0.

5.  The pharmaceutical composition of any one of claims 1-4, wherein the pharmaceutical composition further comprises a stabilizer selected from one or more of arginine hydrochloride, sodium chloride, mannitol, sorbitol, sucrose and trehalose; preferably, the stabilizer is trehalose or sucrose.

6.  The pharmaceutical composition of claim 5, wherein the stabilizer is sodium chloride with a concentration of about 30-200 mM; or the stabilizer is mannitol with a concentration of about 100-300 mM; or the stabilizer is sorbitol with a concentration of about 100-300 mM; or the stabilizer is sucrose with a concentration of about 100-300 mM; or the stabilizer is trehalose with a concentration of about 100-300 mM; or the stabilizer is arginine hydrochloride with a concentration of about 30-200 mM;

    or the stabilizer is a combination of about 30-200 mM sodium chloride and about 30-200 mM mannitol; or the stabilizer is a combination of about 30-200 mM sodium chloride and about 30-200 mM sucrose; or the stabilizer is a combination of about 30-200 mM sodium chloride and about 30-200 mM trehalose;
    preferably, the stabilizer is about 100-300 mM trehalose, 100-300 mM sucrose, about 100-300 mM mannitol or about 100-300 mM sorbitol, and more preferably, about 100-300 mM trehalose.

7. The pharmaceutical composition of any one of claims 1-4, wherein the pharmaceutical composition further comprises a surfactant selected from polysorbate 80, polysorbate 20 or poloxamer 188; preferably, the surfactant is polysorbate 80.

8. The pharmaceutical composition of claim 7, wherein the concentration of the surfactant is about 0.01%-0.1%; preferably, the concentration of the surfactant is about 0.02%-0.08%.

9. The pharmaceutical composition of any one of claims 1-4, wherein the anti-TIGIT antibody comprises:

(I) a heavy chain variable region having an amino acid sequence as represented by SEQ ID NO:7, and a light chain variable region having an amino acid sequence as represented by SEQ ID NO:8; or
(II) a heavy chain variable region having an amino acid sequence as represented by SEQ ID NO:9, and a light chain variable region having an amino acid sequence as represented by SEQ ID NO:10.

10. The pharmaceutical composition of any one of claims 1-4, wherein the anti-TIGIT antibody comprises:

(I) a heavy chain amino acid sequence as represented by SEQ ID NO:11, and a light chain amino acid sequence as represented by SEQ ID NO:12; or
(II) a heavy chain amino acid sequence as represented by SEQ ID NO:13, and a light chain amino acid sequence as represented by SEQ ID NO:14.

11. The pharmaceutical composition of any one of claims 1-4, wherein the anti-TIGIT antibody or the antigen-binding fragment thereof has a concentration of about 1-200 mg/mL, preferably about 10-80 mg/mL, and more preferably about 10-40 mg/mL.

12. The pharmaceutical composition of any one of claims 1-11, comprising the components as shown in any one of the following (1) - (8), or consisting of the components as shown in any one of (1) - (8), respectively:

(1) (a) about 10 mg/mL-80 mg/mL anti-TIGIT antibody or antigen-binding fragment thereof; (b) about 10-30 mM acetate buffer, with a pH of about 5.0; (c) about 100-300 mM sorbitol; and (d) about 0.01%-0.1% polysorbate 80; or
(2) (a) about 10 mg/mL-80 mg/mL anti-TIGIT antibody or antigen-binding fragment thereof; (b) about 10-30 mM acetate buffer, with a pH of about 5.5; (c) about 100-300 mM mannitol; and (d) about 0.01%-0.1% polysorbate 80; or
(3) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM trehalose; and (d) about 0.01%-0.1% polysorbate 80; or
(4) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM sucrose; and (d) about 0.01%-0.1% polysorbate 80; or
(5) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM sucrose; and (d) about 0.01%-0.1% polysorbate 80; or
(6) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM trehalose; and (d) about 0.01%-0.1% polysorbate 80; or
(7) (a) about 10 mg/mL-80 mg/mL anti-TIGIT antibody or antigen-binding fragment thereof; (b) about 10-30 mM acetate buffer, with a pH of about 5.0-6.0; (c) about 100-300 mM trehalose; (d) about 30-200 mM sodium chloride; and (e) about 0.01%-0.1% polysorbate 80; or
(8) (a) about 10 mg/mL-80 mg/mL anti-TIGIT antibody or antigen-binding fragment thereof; (b) about 10-30 mM citrate buffer, with a pH of about 5.0-6.0; (c) about 100-300 mM sucrose; (d) about 30-200 mM sodium chloride; and (e) about 0.01%-0.1% polysorbate 80.

13. The pharmaceutical composition of claim 12, comprising the components as shown in any one of the following (1) - (7), or consisting of the components as shown in any one of (1) - (7), respectively:

(1) (a) about 40 mg/mL anti-TIGIT antibody or antigen-binding fragment thereof; (b) about 20 mM acetate buffer, with a pH of about 5.0; (c) about 220 mM sucrose; and (d) about 0.02% polysorbate 80; or

(2) (a) about 40 mg/mL anti-TIGIT antibody or antigen-binding fragment thereof; (b) about 20 mM acetate buffer, with a pH of about 5.0; (c) about 220 mM trehalose; and (d) about 0.02% polysorbate 80; or

(3) (a) about 40 mg/mL anti-TIGIT antibody or antigen-binding fragment thereof; (b) about 20 mM acetate buffer, with a pH of about 5.5; (c) about 240 mM mannitol; and (d) about 0.02% polysorbate 80; or

(4) (a) about 40 mg/mL anti-TIGIT antibody or antigen-binding fragment thereof; (b) about 20 mM acetate buffer, with a pH of about 5.5; (c) about 220 mM sucrose; and (d) about 0.02% polysorbate 80; or

(5) (a) about 40 mg/mL anti-TIGIT antibody or antigen-binding fragment thereof; (b) about 20 mM acetate buffer, with a pH of about 5.5; (c) about 220 mM trehalose; and (d) about 0.02% polysorbate 80; or

(6) (a) about 40 mg/mL anti-TIGIT antibody or antigen-binding fragment thereof; (b) about 20 mM acetate buffer, with a pH of about 5.5; (c) about 140 mM trehalose; (d) about 50 mM sodium chloride; and (e) about 0.02% polysorbate 80; or

(7) (a) about 40 mg/mL anti-TIGIT antibody or antigen-binding fragment thereof; (b) about 20 mM acetate buffer, with a pH of about 5.5; (c) about 140 mM sucrose; (d) about 50 mM sodium chloride; and (e) about 0.02% polysorbate 80.

14. A liquid preparation or lyophilized preparation, comprising the pharmaceutical composition of any one of claims 1-13.

15. Use of the pharmaceutical composition of any one of claims 1-13 in the preparation of a drug for treating or preventing a TIGIT-mediated disease or condition; preferably, the disease or condition is cancer.

FIG. 1

FIG. 2

FIG. 3

## INTERNATIONAL SEARCH REPORT

| International application No. |
|---|
| **PCT/CN2022/072033** |

| **A.** | **CLASSIFICATION OF SUBJECT MATTER** |
|---|---|
| | C07K 16/28(2006.01)i;　A61P 35/00(2006.01)i;　A61K 39/395(2006.01)i;　C07K 16/18(2006.01)i |

According to International Patent Classification (IPC) or to both national classification and IPC

| **B.** | **FIELDS SEARCHED** |
|---|---|

Minimum documentation searched (classification system followed by classification symbols)

　　　C07K，A61P，A61K，C07K

Documentation searched other than minimum documentation to the extent that such documents are included in the fields searched

Electronic data base consulted during the international search (name of data base and, where practicable, search terms used)

　　　CNABS, DWPI, SIPOABS, CNTXT, WOTXT, JPTXT, USTXT, EPTXT, 万方, CNKI, PubMed, GenBank, ISI web of knowledge: TIGIT, 抗体, antibody, SEQ ID NO: 1-6, 11-14

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| PX | WO 2021008523 A1 (SHANGHAI JUNSHI BIOSCIENCES CO., LTD. et al.) 21 January 2021 (2021-01-21)<br>　　entire document | 1-15 |
| A | CN 110997720 A (ITEOS THERAPEUTICS SA.) 10 April 2020 (2020-04-10)<br>　　claim 1 | 1-15 |
| A | CA 2994555 A1 (MERCK SHARP & DOHME CORP.) 23 February 2017 (2017-02-23)<br>　　entire document | 1-15 |
| A | CN 111744007 A (JIANGSU HENGRUI MEDICINE CO., LTD. et al.) 09 October 2020 (2020-10-09)<br>　　entire document | 1-15 |
| A | WO 2017053748 A2 (GENENTECH, INC. et al.) 30 March 2017 (2017-03-30)<br>　　entire document | 1-15 |
| A | CN 110818795 A (SHANGHAI HENLIUS BIOTECH CO., LTD.) 21 February 2020 (2020-02-21)<br>　　entire document | 1-15 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| | **13 April 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/072033** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.    ☑ forming part of the international application as filed:

         ☑ in the form of an Annex C/ST.25 text file.

         ☐ on paper or in the form of an image file.

     b.    ☐ furnished together with the international application under PCT Rule 13*ter*.1(a) for the purposes of international search only in the form of an Annex C/ST.25 text file.

     c.    ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

| | | International application No. |
|---|---|---|
| | | PCT/CN2022/072033 |

| Patent document cited in search report | | | Publication date (day/month/year) | Patent family member(s) | | | Publication date (day/month/year) |
|---|---|---|---|---|---|---|---|
| WO | 2021008523 | A1 | 21 January 2021 | IL | 289656 | D0 | 01 March 2022 |
| | | | | AU | 2020314129 | A1 | 24 February 2022 |
| | | | | CA | 3146986 | A1 | 21 January 2021 |
| CN | 110997720 | A | 10 April 2020 | US | 2020407445 | A1 | 31 December 2020 |
| | | | | IL | 272227 | D0 | 31 March 2020 |
| | | | | CA | 3070791 | A1 | 31 January 2019 |
| | | | | BR | 112020001499 | A2 | 08 September 2020 |
| | | | | JP | 2020528768 | A | 01 October 2020 |
| | | | | US | 2019100591 | A1 | 04 April 2019 |
| | | | | TW | 201910351 | A | 16 March 2019 |
| | | | | KR | 20200100589 | A | 26 August 2020 |
| | | | | AR | 112768 | A1 | 11 December 2019 |
| | | | | EP | 3719040 | A1 | 07 October 2020 |
| | | | | US | 2019315867 | A1 | 17 October 2019 |
| | | | | SG | 11202000660 Q | A | 27 February 2020 |
| | | | | EP | 3484925 | A1 | 22 May 2019 |
| | | | | ES | 2812236 | T3 | 16 March 2021 |
| | | | | AU | 2018306463 | A1 | 05 March 2020 |
| CA | 2994555 | A1 | 23 February 2017 | EP | 3334757 | A2 | 20 June 2018 |
| | | | | WO | 2017030823 | A2 | 23 February 2017 |
| | | | | CN | 108290936 | A | 17 July 2018 |
| | | | | JP | 2018527919 | A | 27 September 2018 |
| | | | | AU | 2016307845 | A1 | 08 March 2018 |
| | | | | US | 2018371083 | A1 | 27 December 2018 |
| | | | | US | 2021017276 | A1 | 21 January 2021 |
| CN | 111744007 | A | 09 October 2020 | None | | | |
| WO | 2017053748 | A2 | 30 March 2017 | NZ | 739750 | A | 29 November 2019 |
| | | | | CN | 108290946 | A | 17 July 2018 |
| | | | | CL | 2018000744 | A1 | 06 July 2018 |
| | | | | AU | 2019246814 | A1 | 31 October 2019 |
| | | | | BR | 112018005862 | A2 | 16 October 2018 |
| | | | | UA | 124573 | C2 | 13 October 2021 |
| | | | | AU | 2016325610 | A1 | 01 March 2018 |
| | | | | CA | 2994858 | A1 | 30 March 2017 |
| | | | | EP | 3353210 | A2 | 01 August 2018 |
| | | | | MX | 2018003633 | A | 01 August 2018 |
| | | | | CL | 2020000938 | A1 | 14 August 2020 |
| | | | | CR | 20180225 | A | 09 July 2018 |
| | | | | JP | 2020074778 | A | 21 May 2020 |
| | | | | US | 2021253693 | A1 | 19 August 2021 |
| | | | | KR | 20180053742 | A | 23 May 2018 |
| | | | | JP | 2018532397 | A | 08 November 2018 |
| | | | | RU | 2018114523 | A3 | 25 October 2019 |
| | | | | MA | 42925 | A | 01 August 2018 |
| | | | | AU | 2021250946 | A1 | 11 November 2021 |
| | | | | ZA | 201800941 | B | 29 May 2019 |
| | | | | PH | 12018500608 | A1 | 01 October 2018 |
| | | | | SG | 10202007764 T | A | 29 September 2020 |
| | | | | US | 2017088613 | A1 | 30 March 2017 |
| | | | | US | 2018186875 | A1 | 05 July 2018 |

Form PCT/ISA/210 (patent family annex) (January 2015)

**EP 4 293 045 A1**

<table>
<tr><td colspan="2" align="center">INTERNATIONAL SEARCH REPORT<br>Information on patent family members</td><td colspan="2">International application No.<br><br>**PCT/CN2022/072033**</td></tr>
<tr><td align="center">Patent document<br>cited in search report</td><td align="center">Publication date<br>(day/month/year)</td><td align="center">Patent family member(s)</td><td align="center">Publication date<br>(day/month/year)</td></tr>
<tr><td></td><td></td><td>IL     257636   D0</td><td align="center">30 April 2018</td></tr>
<tr><td></td><td></td><td>CO   2018004090  A2</td><td align="center">30 November 2018</td></tr>
<tr><td></td><td></td><td>PE    20181046  A1</td><td align="center">03 July 2018</td></tr>
<tr><td></td><td></td><td>JP   2021166524   A</td><td align="center">21 October 2021</td></tr>
<tr><td></td><td></td><td>US   2021032328  A1</td><td align="center">04 February 2021</td></tr>
<tr><td></td><td></td><td>KR  20200087283  A</td><td align="center">20 July 2020</td></tr>
<tr><td></td><td></td><td>HK    1258058  A1</td><td align="center">01 November 2019</td></tr>
<tr><td></td><td></td><td>US   2022064286  A1</td><td align="center">03 March 2022</td></tr>
<tr><td></td><td></td><td>RU   2020120073   A</td><td align="center">03 July 2020</td></tr>
<tr><td></td><td></td><td>US   2019119376  A1</td><td align="center">25 April 2019</td></tr>
<tr><td></td><td></td><td>TW    201718644   A</td><td align="center">01 June 2017</td></tr>
<tr><td>CN   110818795  A</td><td align="center">21 February 2020</td><td>WO   2021139777  A1</td><td align="center">15 July 2021</td></tr>
</table>

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004024068 A
- WO 2009126688 A
- WO 2015009856 A
- WO 2016028656 A
- CN 2020101883 W
- CN 2013102582892

### Non-patent literature cited in the description

- **STANIETSKY et al.** *PNAS,* 2009, vol. 106, 17858-17863
- **LIU et al.** *Cell death and differentiation,* 2013, vol. 20, 456-464
- **STANIETSKY et al.** *European journal of immunology,* 2013, vol. 43, 2138-2150
- Peptide and Protein Drug Delivery. Marcel Dekker, Inc, 1991, 247-301
- **JONES, A.** *Adv. Drug Delivery Rev.,* 1993, vol. 10, 29-90
- Directorate for the Quality of Medicine of the Council of Europe'' (EDQM). European Pharmacopoeia
- **CHOTHIA et al.** *Nature,* 1989, vol. 342, 877-883
- **AL-LAZIKANI et al.** Standard conformations for the canonical structures of immunoglobulins. *Journal of Molecular Biology,* 1997, vol. 273, 927-948
- **KABAT et al.** Sequences of Proteins of Immunological Interest. U.S. Department of Health and Human Services, National Institutes of Health, 1987
- *Nucleic Acids Research,* 1999, vol. 27, 209-212